# EUROPEAN PATENT APPLICATION

(11) **EP 4 623 714 A1**
(43) Date of publication of application: **01.10.2025**
(21) Application number: 24165920.0
(22) Date of filing: 25.03.2024
(51) Int. Cl.: A24F 15/01, A24F 15/015, A24F 40/48, A61L 2/10

(54) **SYSTEM FOR REFILLING AEROSOL PROVISION DEVICE, DEVICE AND METHOD**

(71) Applicant: Nicoventures Trading Limited, London WC2R 3LA (GB)
(72) Inventor: BISHOP, David, London, WC2R 3LA (GB); ROTHWELL, Howard, London, WC2R 3LA (GB); YILMAZ, Ugurhan, London, WC2R 3LA (GB); DANIELS, Christopher, London, WC2R 3LA (GB); WILLIAMS, Keiann, London, WC2R 3LA (GB); HARRIMAN, Mark, London, WC2R 3LA (GB); OHENE, Andrews, London, WC2R 3LA (GB)
(74) Representative: D Young & Co LLP

(57) **Abstract**

Described is a system including: an aerosol provision device comprising an aerosol-generating material storage portion for storing an aerosol-generating material, the aerosol provision device arranged to aerosolise aerosol-generating material stored in the aerosol-generating material storage portion, and a refill device interface fluidly coupled to the aerosol-generating material storage portion; a refilling device comprising a transfer mechanism for transferring aerosol-generating material from the refilling device to the aerosol-generating material storage portion when the aerosol provision device is coupled to the refilling device, and an aerosol provision device interface fluidly coupled to a source of aerosol-generating material of the refilling device; and a sterilisation component. The aerosol provision device is configured to couple to the refilling device via engagement of the refill device interface with the aerosol provision device interface such that aerosol-generating material is capable of being transferred to the aerosol-generating material storage portion from the refilling device via the coupled interfaces. The sterilisation component is configured to prevent or reduce microbial growth at at least one of the refill device interface and the aerosol provision device interface. Also described is an aerosol provision device, a refilling device and a method for reducing or preventing microbial growth.

## Description

### Field

The present disclosure relates to systems for providing aerosol to a user including electronic aerosol provision systems such as nicotine delivery systems (e.g. electronic cigarettes and the like).

### Background

Electronic aerosol provision systems such as electronic cigarettes (e-cigarettes) are used by some as a replacement or alternative to cigarettes, for example such as those looking to reduce nicotine consumption.

Electronic aerosol provision systems such as electronic cigarettes (e-cigarettes) generally contain a reservoir of a source liquid containing a formulation, typically including nicotine, from which an aerosol is generated, e.g. through heat vaporisation. An aerosol source for an aerosol provision system may thus comprise a heater having a heating element arranged to receive source liquid from the reservoir, for example through wicking / capillary action. While a user inhales on the device, electrical power is supplied to the heating element to vaporise source liquid in the vicinity of the heating element to generate an aerosol for inhalation by the user. Such devices are usually provided with one or more air inlet holes located away from a mouthpiece end of the system. When a user sucks on a mouthpiece connected to the mouthpiece end of the system, air is drawn in through the inlet holes and past the aerosol source. There is a flow path connecting between the aerosol source and an opening in the mouthpiece so that air drawn past the aerosol source continues along the flow path to the mouthpiece opening, carrying some of the aerosol from the aerosol source with it. The aerosol-carrying air exits the aerosol provision system through the mouthpiece opening for inhalation by the user.

To help reduce material wastage and/or disposal of such electronic aerosol provision systems, some electronic aerosol provision systems are provided with refillable reservoirs, which can be refilled with aerosol-generating material and used for multiple times without disposal. However, the refilling process for such refillable aerosol provision systems can be difficult, and can be prone to leakage of aerosol-generating material as well as providing exposure of certain components of the refillable aerosol provision system to the environment. This may lead to microbial growth in certain areas of the system which may impact consumer experiences (e.g., foul smells or tastes, etc.)

Various approaches are described which seek to help address some of these issues.

### Summary

According to a first aspect of certain embodiments there is provided a system including: an aerosol provision device comprising an aerosol-generating material storage portion for storing an aerosol-generating material, the aerosol provision device arranged to aerosolise aerosol-generating material stored in the aerosol-generating material storage portion, and a refill device interface fluidly coupled to the aerosol-generating material storage portion; a refilling device comprising a transfer mechanism for transferring aerosol-generating material from the refilling device to the aerosol-generating material storage portion when the aerosol provision device is coupled to the refilling device, and an aerosol provision device interface fluidly coupled to a source of aerosol-generating material of the refilling device; and a sterilisation component. The aerosol provision device is configured to couple to the refilling device via engagement of the refill device interface with the aerosol provision device interface such that aerosol-generating material is capable of being transferred to the aerosol-generating material storage portion from the refilling device via the coupled interfaces. The sterilisation component is configured to prevent or reduce microbial growth at at least one of the refill device interface and the aerosol provision device interface.

In some examples, the sterilisation component comprises a UV light emitter arranged to direct UV light towards the at least one of the refill device interface and the aerosol provision device interface.

In some examples, the UV light emitter is provided as part of the refilling device.

In some examples, the refilling device comprises an aerosol provision device receiving section configured to receive at least a part of the aerosol provision device therein, and wherein the UV light emitter is arranged to direct light into the aerosol provision device receiving section.

In some examples, the UV light emitter is configured to be operated in at least one of: when the aerosol provision device is provided in the aerosol provision device receiving section; when the refilling device is controlled to perform a refilling operation; and when the aerosol provision device is removed from the aerosol provision device receiving section.

In some examples, the UV light emitter is configured to be operated when a user engages with a UV light emitter activation button provided on at least one of the refilling device and the aerosol provision device.

In some examples, the sterilisation component comprises a coating, the coating provided on at least one of the refill device interface and the aerosol provision device interface.

In some examples, the coating is an anti-microbial growth coating, and wherein optionally the coating is, or comprises, silver.

In some examples, the sterilisation component is further configured to prevent or reduce microbial growth at or in the aerosol-generating material storage portion of the aerosol provision device.

In some examples, the sterilisation component is further configured to prevent or reduce microbial growth at or in an aerosol-generating material storage portion of the refilling device.

In some examples, the system further comprises aerosol-generating material that is free from active ingredients and / or flavourants.

According to a second aspect of certain embodiments there is provided an aerosol provision device for aerosolising aerosol-generating material, the aerosol provision device including: an aerosol-generating material storage portion for storing an aerosol-generating material, a refill device interface fluidly coupled to the aerosol-generating material storage portion and arranged to engage with an aerosol provision device interface of a refilling device, the refilling device comprising a transfer mechanism for transferring aerosol-generating material from the refilling device to the aerosol-generating material storage portion when the aerosol provision device is coupled to the refilling device; and a sterilisation component. The aerosol provision device is configured to couple to the refilling device via engagement of the refill device interface with the aerosol provision device interface such that aerosol-generating material is capable of being transferred to the aerosol-generating material storage portion from the refilling device via the coupled interfaces. The sterilisation component is configured to prevent or reduce microbial growth at least the refill device interface.

According to a third aspect of certain embodiments there is provided a refilling device for refilling an aerosol-generating material storage portion of an aerosol provision device arranged to aerosolise aerosol-generating material stored in the aerosol-generating material storage portion, the refilling device including: a transfer mechanism for transferring aerosol-generating material from the refilling device to the aerosol-generating material storage portion when the aerosol provision device is coupled to the refilling device, an aerosol provision device interface fluidly coupled to a source of aerosol-generating material of the refilling device and configured to engage with a refill device interface of the aerosol provision device; and a sterilisation component. The refilling device is configured to couple to the aerosol provision device via engagement of the aerosol provision device interface with the refill device interface such that aerosol-generating material is capable of being transferred to the aerosol-generating material storage portion from the refilling device via the coupled interfaces. The sterilisation component is configured to prevent or reduce microbial growth at at least the aerosol provision device interface.

According to a fourth aspect of certain embodiments there is provided a method for reducing or preventing microbial growth at at least one of a refill device interface of an aerosol provision device and the aerosol provision device interface of a refilling device, wherein the refilling device is configured to refill an aerosol-generating material storage portion of the aerosol provision device with aerosol generating material via engagement of the refill device interface with the aerosol provision device interface, the method including: providing a sterilisation component at at least one of the refill device interface and the aerosol provision device interface.

According to a fifth aspect of certain embodiments there is provided a system including: aerosol provision means comprising an aerosol-generating material storage means for storing an aerosol-generating material, the aerosol provision means arranged to aerosolise aerosol-generating material stored in the aerosol-generating material storage means, and refill interface means fluidly coupled to the aerosol-generating material storage means; refilling means comprising transfer means for transferring aerosol-generating material from the refilling means to the aerosol-generating material storage means when the aerosol provision means is coupled to the refilling means, and an aerosol provision device interface means fluidly coupled to a source of aerosol-generating material of the refilling means; and sterilisation means. The aerosol provision means is configured to couple to the refilling means via engagement of the refill device interface means with the aerosol provision device interface means such that aerosol-generating material is capable of being transferred to the aerosol-generating material storage means from the refilling means via the coupled interface means. The sterilisation means is configured to prevent or reduce microbial growth at at least one of the refill device interface means and the aerosol provision device interface means.

It will be appreciated that features and aspects of the invention described above in relation to the first and other aspects of the invention are equally applicable to, and may be combined with, embodiments of the invention according to other aspects of the invention as appropriate, and not just in the specific combinations described above.

### Brief Description of the Drawings

Embodiments of the invention will now be described, by way of example only, with reference to the accompanying drawings, in which:
Figure 1 schematically shows a system for providing an aerosol to a user, including an aerosol provision device, an aerosol modifying agent release component, and a refill and recharge device according to an aspect of the present disclosure;
Figure 2 schematically shows, in cross-section, the aerosol provision device of the system of Figure 1 in more detail;
Figure 3 schematically shows, in cross-section, the consumable of the system of Figure 1 in more detail;
Figure 4 schematically shows, in cross-section, the refill and recharge device of the system of Figure 1 in more detail;
Figure 5a schematically shows, in cross-section, a section of the refill and recharge device of Figure 4 showing an active sterilisation component arranged in the refill and recharge device in accordance with the present disclosure;
Figure 5b schematically shows, in cross-section, a section of the refill and recharge device of Figure 4 showing an alternative arrangement of the active sterilisation component in the refill and recharge device compared to Figure 5a in accordance with the present disclosure;
Figure 5c schematically shows, in cross-section, a section of the refill and recharge device of Figure 4 showing an active sterilisation component arranged in the aerosol provision device in accordance with the present disclosure;
Figure 6 schematically shows, in cross-section, a section of the refill and recharge device of Figure 4 showing passive sterilisations components arranged in the refill and recharge device and the aerosol provision device in accordance with the present disclosure;
Figure 7 schematically shows, in cross-section, a modification of the refill and recharge device of Figure 4 comprising an active sterilisation component arranged to reduce or prevent microbial growth at a reservoir of the refill/recharge device;
Figure 8 schematically shows, in cross-section, a modification of the aerosol provision device of Figure 2 comprising a passive sterilisation component arranged to reduce or prevent microbial growth at a reservoir of the aerosol provision device;
Figure 9 schematically shows, in cross-section, a modification of the refill and recharge device of the system of Figure 1 in more detail, where the refill and recharge device comprises a lid; and
Figure 10 shows a flow chart depicting a method for reducing or preventing microbial growth at at least one of a refill and recharge device interface of an aerosol provision device and the aerosol provision device interface of a refill and recharge device according to an aspect of the present disclosure.

### Detailed Description

Aspects and features of certain examples and embodiments are discussed / described herein. Some aspects and features of certain examples and embodiments may be implemented conventionally and these are not discussed / described in detail in the interests of brevity. It will thus be appreciated that aspects and features of apparatus and methods discussed herein which are not described in detail may be implemented in accordance with any conventional techniques for implementing such aspects and features.

According to the present disclosure, a "non-combustible" aerosol provision system is one where a constituent aerosol-generating material of the aerosol provision system (or component thereof) is not combusted or burned in order to facilitate delivery of at least one substance to a user.

In some embodiments, the delivery system is a non-combustible aerosol provision system, such as a powered non-combustible aerosol provision system.

In some embodiments, the non-combustible aerosol provision system is an electronic cigarette, also known as a vaping device or electronic nicotine delivery system (END), although it is noted that the presence of nicotine in the aerosol-generating material is not a requirement.

In some embodiments, the non-combustible aerosol provision system is a hybrid system to generate aerosol using a combination of aerosol-generating materials, one or a plurality of which may be heated. Each of the aerosol-generating materials may be, for example, in the form of a solid, liquid or gel and may or may not contain nicotine. In some embodiments, the hybrid system comprises a liquid or gel aerosol-generating material and a solid aerosol-generating material. The solid aerosol-generating material may comprise, for example, tobacco or a non-tobacco product.

Typically, the non-combustible aerosol provision system may comprise a non-combustible aerosol provision device and a consumable for use with the non-combustible aerosol provision device.

In some embodiments, the disclosure relates to consumables comprising aerosol-generating material and configured to be used with non-combustible aerosol provision devices. These consumables are sometimes referred to as articles throughout the disclosure.

In some embodiments, the non-combustible aerosol provision system, such as a non-combustible aerosol provision device thereof, may comprise a power source and a controller. The power source may, for example, be an electric power source.

In some embodiments, the non-combustible aerosol provision system may comprise an area for receiving the consumable, an aerosol generator, an aerosol generation area, a housing, a mouthpiece, a filter and/or an aerosol-modifying agent.

In some embodiments, the consumable for use with the non-combustible aerosol provision device may comprise aerosol-generating material, an aerosol-generating material storage area, an aerosol-generating material transfer component, an aerosol generation area, a housing, a wrapper, a filter, a mouthpiece, and/or an aerosol-modifying agent.

In some embodiments, the substance to be delivered may be an aerosol-generating material. As appropriate, either material may comprise one or more active constituents, one or more flavours, one or more aerosol-former materials, and/or one or more other functional materials.

Aerosol-generating material is a material that is capable of generating aerosol, for example when heated, irradiated or energized in any other way. Aerosol-generating material may, for example, be in the form of a solid, liquid or gel which may or may not contain an active substance and/or flavourants. In some embodiments, the aerosol-generating material may comprise an "amorphous solid", which may alternatively be referred to as a "monolithic solid" (i.e. non-fibrous). In some embodiments, the amorphous solid may be a dried gel. The amorphous solid is a solid material that may retain some fluid, such as liquid, within it. In some embodiments, the aerosol-generating material may for example comprise from about 50wt%, 60wt% or 70wt% of amorphous solid, to about 90wt%, 95wt% or 100wt% of amorphous solid.

The aerosol-generating material may comprise one or more active substances and/or flavours, one or more aerosol-former materials, and optionally one or more other functional material.

An aerosol-modifying agent is a substance, typically located downstream of the aerosol generation area, that is configured to modify the aerosol generated, for example by changing the taste, flavour, acidity or another characteristic of the aerosol. The aerosol-modifying agent may be provided in an aerosol-modifying agent release component, that is operable to selectively release the aerosol-modifying agent. The aerosol-modifying agent may, for example, be an additive or a sorbent. The aerosol-modifying agent may, for example, comprise one or more of an active substance, a flavourant, a colourant, water, and a carbon adsorbent. The aerosol-modifying agent may, for example, be a solid, a liquid, or a gel. The aerosol-modifying agent may be in powder, thread or granule form. The aerosol-modifying agent may be free from filtration material.

The aerosol-former material may comprise one or more constituents capable of forming an aerosol. In some embodiments, the aerosol-former material may comprise one or more of glycerol, propylene glycol, diethylene glycol, triethylene glycol, tetraethylene glycol, 1,3-butylene glycol, erythritol, meso-Erythritol, ethyl vanillate, ethyl laurate, a diethyl suberate, triethyl citrate, triacetin, a diacetin mixture, benzyl benzoate, benzyl phenyl acetate, tributyrin, lauryl acetate, lauric acid, myristic acid, and propylene carbonate.

In some embodiments, the aerosol-generating material and/or the aerosol-modifying agent comprises an active substance.

The active substance as used herein may be a physiologically active material, which is a material intended to achieve or enhance a physiological response. The active substance may for example be selected from nutraceuticals, nootropics, psychoactives. The active substance may be naturally occurring or synthetically obtained. The active substance may comprise for example nicotine, caffeine, taurine, theine, vitamins such as B6 or B12 or C, melatonin, cannabinoids, or constituents, derivatives, or combinations thereof. The active substance may comprise one or more constituents, derivatives or extracts of tobacco, cannabis or another botanical.

In some embodiments, the active substance comprises nicotine. In some embodiments, the active substance comprises caffeine, melatonin or vitamin B12.

As noted herein, the active substance may comprise one or more constituents, derivatives or extracts of cannabis, such as one or more cannabinoids or terpenes.

As noted herein, the active substance may comprise or be derived from one or more botanicals or constituents, derivatives or extracts thereof. As used herein, the term "botanical" includes any material derived from plants including, but not limited to, extracts, leaves, bark, fibres, stems, roots, seeds, flowers, fruits, pollen, husk, shells or the like. Alternatively, the material may comprise an active compound naturally existing in a botanical, obtained synthetically. The material may be in the form of liquid, gas, solid, powder, dust, crushed particles, granules, pellets, shreds, strips, sheets, or the like. Example botanicals are tobacco, eucalyptus, star anise, hemp, cocoa, cannabis, fennel, lemongrass, peppermint, spearmint, rooibos, chamomile, flax, ginger, ginkgo biloba, hazel, hibiscus, laurel, licorice (liquorice), matcha, mate, orange skin, papaya, rose, sage, tea such as green tea or black tea, thyme, clove, cinnamon, coffee, aniseed (anise), basil, bay leaves, cardamom, coriander, cumin, nutmeg, oregano, paprika, rosemary, saffron, lavender, lemon peel, mint, juniper, elderflower, vanilla, wintergreen, beefsteak plant, curcuma, turmeric, sandalwood, cilantro, bergamot, orange blossom, myrtle, cassis, valerian, pimento, mace, damien, marjoram, olive, lemon balm, lemon basil, chive, carvi, verbena, tarragon, geranium, mulberry, ginseng, theanine, theacrine, maca, ashwagandha, damiana, guarana, chlorophyll, baobab or any combination thereof. The mint may be chosen from the following mint varieties: Mentha Arventis, Mentha c.v.,Mentha niliaca, Mentha piperita, Mentha piperita citrata c.v.,Mentha piperita c.v, Mentha spicata crispa, Mentha cardifolia, Memtha longifolia, Mentha suaveolens variegata, Mentha pulegium, Mentha spicata c.v. and Mentha suaveolens

In some embodiments, the active substance comprises or is derived from one or more botanicals or constituents, derivatives or extracts thereof and the botanical is tobacco.

In some embodiments, the active substance comprises or derived from one or more botanicals or constituents, derivatives or extracts thereof and the botanical is selected from eucalyptus, star anise, cocoa and hemp.

In some embodiments, the active substance comprises or derived from one or more botanicals or constituents, derivatives or extracts thereof and the botanical is selected from rooibos and fennel.

In some embodiments, the aerosol-generating material and/or aerosol-modifying agent comprises a flavour.

As used herein, the terms "flavour" and "flavourant" refer to materials which, where local regulations permit, may be used to create a desired taste, aroma or other somatosensorial sensation in a product for adult consumers. They may include naturally occurring flavour materials, botanicals, extracts of botanicals, synthetically obtained materials, or combinations thereof (e.g., tobacco, cannabis, licorice (liquorice), hydrangea, eugenol, Japanese white bark magnolia leaf, chamomile, fenugreek, clove, maple, matcha, menthol, Japanese mint, aniseed (anise), cinnamon, turmeric, Indian spices, Asian spices, herb, wintergreen, cherry, berry, red berry, cranberry, peach, apple, orange, mango, clementine, lemon, lime, tropical fruit, papaya, rhubarb, grape, durian, dragon fruit, cucumber, blueberry, mulberry, citrus fruits, Drambuie, bourbon, scotch, whiskey, gin, tequila, rum, spearmint, peppermint, lavender, aloe vera, cardamom, celery, cascarilla, nutmeg, sandalwood, bergamot, geranium, khat, naswar, betel, shisha, pine, honey essence, rose oil, vanilla, lemon oil, orange oil, orange blossom, cherry blossom, cassia, caraway, cognac, jasmine, ylang-ylang, sage, fennel, wasabi, piment, ginger, coriander, coffee, hemp, a mint oil from any species of the genus Mentha, eucalyptus, star anise, cocoa, lemongrass, rooibos, flax, ginkgo biloba, hazel, hibiscus, laurel, mate, orange skin, rose, tea such as green tea or black tea, thyme, juniper, elderflower, basil, bay leaves, cumin, oregano, paprika, rosemary, saffron, lemon peel, mint, beefsteak plant, curcuma, cilantro, myrtle, cassis, valerian, pimento, mace, damien, marjoram, olive, lemon balm, lemon basil, chive, carvi, verbena, tarragon, limonene, thymol, camphene), flavour enhancers, bitterness receptor site blockers, sensorial receptor site activators or stimulators, sugars and/or sugar substitutes (e.g., sucralose, acesulfame potassium, aspartame, saccharine, cyclamates, lactose, sucrose, glucose, fructose, sorbitol, or mannitol), and other additives such as charcoal, chlorophyll, minerals, botanicals, or breath freshening agents. They may be imitation, synthetic or natural ingredients or blends thereof. They may be in any suitable form, for example, liquid such as an oil, solid such as a powder, or gas.

In some embodiments, the flavour comprises menthol, spearmint and/or peppermint. In some embodiments, the flavour comprises flavour components of cucumber, blueberry, citrus fruits and/or redberry. In some embodiments, the flavour comprises eugenol. In some embodiments, the flavour comprises flavour components extracted from tobacco.

In some embodiments, the flavour comprises flavour components extracted from cannabis.

In some embodiments, the flavour may comprise a sensate, which is intended to achieve a somatosensorial sensation which are usually chemically induced and perceived by the stimulation of the fifth cranial nerve (trigeminal nerve), in addition to or in place of aroma or taste nerves, and these may include agents providing heating, cooling, tingling, numbing effect. A suitable heat effect agent may be, but is not limited to, vanillyl ethyl ether and a suitable cooling agent may be, but not limited to eucolyptol, WS-3.

In some embodiments, the aerosol-generating material and/or aerosol-modifying agent comprises an aerosol-former material. Examples of aerosol-former materials are provided above. In some embodiments, the aerosol-generating material and/or aerosol-modifying agent comprises one or more other functional materials, which may comprise one or more of pH regulators, colouring agents, preservatives, binders, fillers, stabilizers, and/or antioxidants.

An aerosol generator is an apparatus configured to cause aerosol to be generated from the aerosol-generating material. In some embodiments, the aerosol generator is a heater configured to subject the aerosol-generating material to heat energy, so as to release one or more volatiles from the aerosol-generating material to form an aerosol. In some embodiments, the aerosol generator is configured to cause an aerosol to be generated from the aerosol-generating material without heating. For example, the aerosol generator may be configured to subject the aerosol-generating material to one or more of vibration, increased pressure, or electrostatic energy.

A susceptor is a material that is heatable by penetration with a varying magnetic field, such as an alternating magnetic field. The susceptor may be an electrically-conductive material, so that penetration thereof with a varying magnetic field causes induction heating of the heating material. The heating material may be magnetic material, so that penetration thereof with a varying magnetic field causes magnetic hysteresis heating of the heating material. The susceptor may be both electrically-conductive and magnetic, so that the susceptor is heatable by both heating mechanisms. The device that is configured to generate the varying magnetic field is referred to as a magnetic field generator, herein.

In accordance with the present disclosure, provided is a system having an aerosol provision device and a refill/recharge pack for, at least, refilling an aerosol-generating material storage portion with aerosol-generating material. The aerosol provision device and the refill/recharge pack are configured to engage with one another to allow aerosol-generating material to be transferred from the refill/recharge pack to the aerosol provision device. The aerosol provision device may be said to have a refill/recharge pack interface that is fluidly coupled to the aerosol-generating material storage portion, while the refill/recharge pack may be said to have an aerosol provision device interface fluidly coupled to a source of aerosol-generating material of the refill/recharge pack. When the aerosol provision device is coupled to the refill/recharge pack for refilling, the two interfaces are brought into fluid communication. The system further comprises a sterilisation component that is configured to prevent or reduce microbial growth at at least one of the refill/recharge pack interface and the aerosol provision device interface. In this way, despite the interfaces being prone to contact with aerosol-generating material and the environment (e.g., air), the sterilisation component can help reduce or prevent microbial growth which may lead to unsatisfactory user experiences and, in some cases, even unhygienic devices.

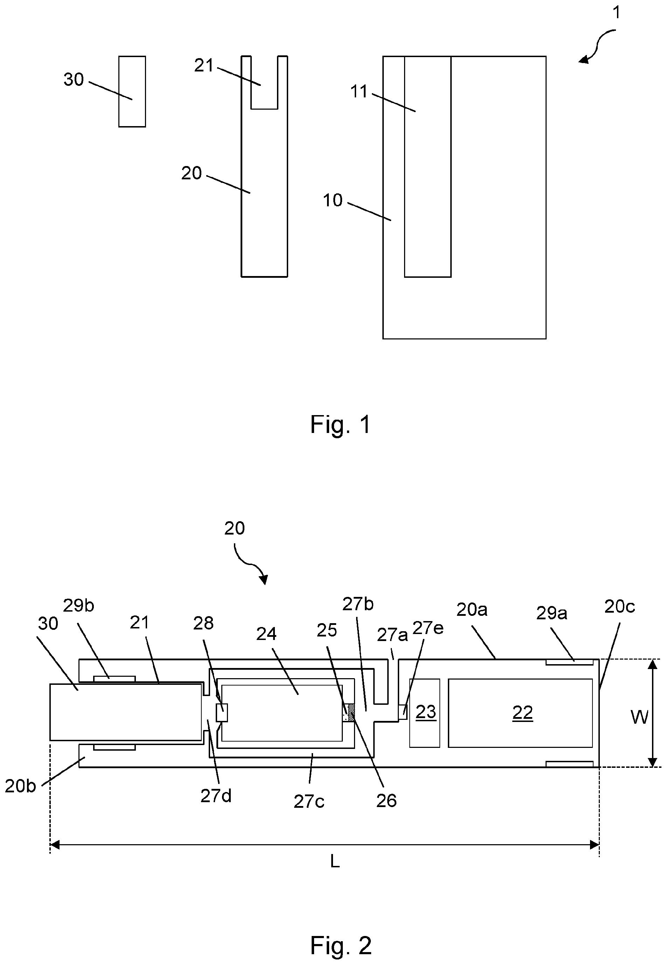

Figure 1 schematically shows a system 1 for providing an aerosol to a user in accordance with aspects of the present disclosure. The system 1 comprises a refill and recharge device 10 (sometimes referred to herein as a refill/recharge pack 10), an aerosol provision device 20, and an aerosol modifying agent release component 30 (sometimes referred to herein as a consumable or article).

Figure 1 schematically shows each of these components separated from one another. Each of these components will be described in more detail below. However, by way of summary, the aerosol provision device 20 is arranged to engage with the consumable 30 (for example, the aerosol provision device 20 may comprise a receptacle 21 for receiving at least a part of the consumable 30), with the consumable 30 and aerosol provision device 20 together being operable to deliver aerosol to a user. The combination of the aerosol provision device 20 and consumable 30 may be referred to herein as an aerosol provision system. The refill/recharge pack 10 is arranged to engage with the aerosol provision device 20 (for example, the refill/recharge pack 10 may comprise a receptacle 11 for receiving at least a part of the aerosol provision device 20), with the refill/recharge pack 10 being operable to recharge and/or refill the aerosol provision device 20 when the aerosol provision device 20 is engaged with the refill/recharge pack 10.

Figure 2 schematically shows the aerosol provision device 20 in more detail, along with a schematic representation of the consumable 30, in accordance with an aspect of the present disclosure. It should be appreciated that Figure 2 is not shown to any particular scale and the various components are only schematically shown. In addition, it should be appreciated that certain features of the aerosol provision device 20 are omitted from Figure 2, such as the various wiring and electrical connections between certain components, for example.

The aerosol provision device 20 comprises a housing 20a, the receptacle 21 (which in this example is formed by the housing 20a), a power source 22, control circuitry 23, an aerosol-generating material storage area (or herein referred to as a reservoir) 24, an aerosol-generating material transport element 25, an aerosol generator 26, an airflow path formed of an air inlet 27a, a vapour generation chamber 27b, air passage 27c, and outlet 27d, a reservoir refill mechanism 28 and electrical contacts 29a and 29b.

In the described example, the aerosol provision system (i.e., the combination of the consumable 30 and the aerosol provision device 20) is configured to have the shape and dimensions of a cigarette. That is, the aerosol provision system may be broadly cylindrical and have a total dimension in the length direction L (i.e., along a longitudinal axis thereof) of between 13 cm and 6 cm, or between 12 cm and 7 cm, or between 9 cm and 8 cm and a total width dimension W (i.e., perpendicular to the longitudinal axis) of between 5 to 10 mm, or between 7 to 9 mm. However, it should be appreciated that in other implementations, the size and/or shape of the aerosol provision system may be different.

With reference to Figure 2, it can be seen that the width of consumable 30 is less than the width of the aerosol provision device 20, thereby providing a stepped profile at the relevant end of the aerosol provision system where the consumable 30 protrudes from the receptacle 21. In some implementations, the consumable 30 may be configured such that the protruding section has a similar width to the aerosol provision device 20. When the two are engaged, the protruding section of the consumable 30 protruding from the receptacle 21 forms a flush outer surface with the outer surface of the aerosol provision device 20. In other implementations, the consumable 30 may couple to the aerosol provision device 20 in a different manner, and may have a width dimension the same as the width dimension of the aerosol provision device 20 (for example, when the consumable 30 is attached at an end of the aerosol provision device 20).

In the described example, the aerosol provision system is intended to be held by a user during use and used in a similar manner to a cigarette. In this regard, providing the aerosol provision system with similar dimensions to a cigarette increases familiarity to users transitioning from cigarettes to electronic aerosol provision systems, which therefore may help ease such a transition. In some implementations, the aerosol provision system may also have a similar weight to a cigarette, for broadly similar reasons.

The outer housing 20a in the described implementation has an overall cylindrical shape. The outer housing 20a defines a proximal or mouth end 20b at which the receptacle 21 and consumable 30 (when present) are located and a distal end 20c, opposite the proximal end 20b. The outer housing 20a may be formed, for example, from a plastics or metallic material. In some implementations, the outer housing 20a may be circumscribed, at least partly, by a paper material or cellulose material. Within the outer housing 20a is located the various components of the aerosol provision device 20, such as the power source 22, control circuitry 23, etc.

The power source 22 in this implementation is a battery 22. The battery 22 is rechargeable and may be, for example, of the kind normally used in aerosol provision systems and other applications requiring provision of relatively high currents over relatively short periods. The battery 22 may be, for example, a lithium ion battery, although other battery chemistries may also be considered. The battery 22 is capable of being recharged via an external source (such as the refill/recharge pack 10, described later). In the present example, the aerosol provision device 20 includes a first electrical contact 29a at the distal end 20c of the outer housing 20a and a second electrical contact 29b at the proximal end 20b of the outer housing 20, positioned at an inner surface of the receptacle 21. The first and second electrical contacts 29a, 29b may be annular and extend around the outer surface of the outer housing 20a and the inner surface of the receptacle 21, accordingly. Broadly, the first electrical contact 29a is configured to couple to a positive terminal of an external power source (or alternatively a negative terminal) and the second electrical contact 29b is configured to couple to a negative terminal of an external power source (or alternatively a positive terminal). The electrical contacts 29a, 29b are electrically coupled to the terminals of the battery 22 (either directly or via suitable recharging circuitry) and can facilitate recharging of the battery 22. However, in other implementations, the electrical contacts 29a, 29b may be arranged differently (for example, at the same end of the housing 20a), or may be omitted if, for example, the battery 22 is to be recharged inductively using suitable wireless recharging circuitry provided in the housing 20a.

The control circuitry 23 is suitably configured / programmed to control the operations of the aerosol provision system. The control circuitry 23 may be considered to logically comprise various sub-units / circuitry elements associated with different aspects of the aerosol provision system's operation and may be implemented by provision of a (micro)controller, processor, ASIC or similar form of control chip. The control circuitry 23 may be arranged to control any functionality associated with the aerosol provision system. By way of non-limiting examples only, the functionality may include the charging or re-charging of the battery 22, the discharging of the battery 22 (e.g., for providing power to the aerosol generator 26), in addition to other functionality such as controlling visual indicators (e.g., LEDs) / displays, communication functionality for communicating with external devices, etc. The control circuitry 23 may be mounted to a printed circuit board (PCB). Note also that the functionality provided by the control circuitry 23 may be split across multiple circuit boards and / or across components which are not mounted to a PCB, and these additional components and / or PCBs can be located as appropriate within the aerosol provision device 20. For example, functionality of the control circuitry 23 for controlling the (re)charging functionality of the battery 23 may be provided separately (e.g. on a different PCB) from the functionality for controlling the discharge of the battery 22.

In the described implementation, the reservoir 24 (which is an example of an aerosol-generating material storage area) is provided as part of the aerosol provision device 20, for example, within housing 20a. In the described implementation, the reservoir 24 may be integrally formed with the aerosol provision device 20. The reservoir 24 may be unable to be removed from the aerosol provision device 20. However, in other implementations, the reservoir 24 may be removable from the aerosol provision device 20 (e.g., provided as a separate but connectable component of the aerosol provision device 20). The reservoir 24 may comprise one or more walls that define a volume, within which an aerosol generating material is capable of being stored. In the present example, the reservoir 24 is configured to store a liquid aerosol-generating material, and may therefore be configured so as to reduce or prevent leakage of the aerosol-generating material out of the reservoir 24. The reservoir 24 may take any suitable shape, such as a cylindrical shape.

In the present example, at one end of the reservoir 24 (e.g., an end closest to the distal end 20c of the housing 20a), the reservoir 24 is provided in fluid communication with an aerosol-generating material transport element 25 and an aerosol generator 26. For example, the reservoir 24 may comprising an opening, within which the aerosol-generating material transport element 25 is located or extends.

The aerosol-generating material transport element 25 is configured to transport the aerosol-generating material from the reservoir 24 to the aerosol generator 26. The specific mechanism underlying how the aerosol-generating material transport element 25 functions may depend on the aerosol-generating material stored in the reservoir 24. In some implementations, where the aerosol-generating material is a liquid or other material capable of flowing, the aerosol-generating material transport element 25 may be configured to transport aerosol-generating material via capillary action. In some implementations, the aerosol-generating material transport element 25 may comprise a porous material (e.g., ceramic) or a bundle of fibres (e.g., glass or cotton fibres) which define a plurality of pores or interstices capable of drawing liquid from the reservoir 24 to be provided to the aerosol generator 26. Any suitable aerosol-generating material transport element 25 may be utilised having regard to the specific aerosol-generating material stored in the reservoir 24 and/or the type of aerosol generator 26 used.

The aerosol generator 26 is an apparatus configured to cause aerosol to be generated from the aerosol-generating material. In the described implementations, the aerosol generator 26 is a heater or heating element. The heating element is configured to subject the aerosol-generating material to heat energy, so as to release one or more volatiles from the aerosol-generating material to form an aerosol. The form of the heating element is not particularly limited. In some implementations, the heating element may be a planar structure. By way of example, the heating element may take the form of an electrically conductive plate or sheet, for example of titanium or other electrically resistive material such as NiChrome. However, in other implementations, the heating element may take other forms, such as an electrically resistive wire or trace or the like. The precise form of the heating element is not specific to the present disclosure; however, it is noted that some technologies may, currently, be more suited to implementation in the aerosol provision device 20 having the size requirements specified above. In some implementations, the heating element is electrically connected to the control circuitry 23 and battery 22, and heating of the heating element is achieved by passing an electrical current between locations on the heating element. In other implementations, the heating element may be a susceptor element which is intended to generate heat upon exposure to an alternating magnetic field (generated by a suitable magnetic field generator located in the aerosol provision device 20 and controlled by the control circuitry 23).

In the described implementation, the aerosol generator 26 is a heating element. However, in yet other implementations, the aerosol generator 26 may be configured to cause an aerosol to be generated from the aerosol-generating material without heating. For example, the aerosol generator 26 may be configured to subject the aerosol-generating material to one or more of vibration, increased pressure, or electrostatic energy.

In the described implementation, the aerosol generator 26 is integrally formed with the aerosol provision device 20. However, in other implementations, the aerosol generator 26 and/or the aerosol-generating material transport element 25 may be removable from the aerosol provision device 20. In implementations where the reservoir 24 is removable, the aerosol generator 26 and aerosol-generating material transport element 25 may be removable with the reservoir 24 (for example, the reservoir 24, aerosol-generating material transport element 25, and aerosol generator 26 may form a single component that is replaceable).

In some implementations, the aerosol-generating material transport component 25 may be integrated with the aerosol generator 26 to form a combined aerosol generator and aerosol-generating material transport component. For example, in some implementations, the aerosol generator 26 may comprise a porous, conductive plate or a plurality of sintered steel fibres forming a planar structure.

In other implementations, the aerosol-generating material transport component 25 may be omitted; for example, in configurations where the aerosol-generating material in the reservoir 24 is capable of being transported to the aerosol generator 26 without the aerosol-generating material transport component 25. However, it should also be acknowledged that the aerosol-generating material transport component 25 may be provided not only to facilitate transport of aerosol-generating material to the aerosol generator 26, but also to regulate the transport of aerosol-generating material to the aerosol generator 26 (e.g., to provide a defined flow of aerosol-generating material).

The aerosol provision device 20 further comprises an airflow path formed of an air inlet 27a, a vapour generation chamber 27b, air passage 27c, and outlet 27d. Optionally, a pressure sensor 27e (or other suitable sensor) is provided in the airflow path. The air inlet 27a comprises one or more openings provided in the outer housing 20a. The air inlet 27a is provided in fluid communication with a vapour generation chamber 27b, which is a region around the aerosol generator 26 where vapour is initially generated and aerosol initially formed. In Figure 2, the vapour generation chamber 27b is shown as region extending the width of the reservoir 24, but it should be appreciated the vapour generation chamber 27b may take any suitable size or shape. The vapour generation chamber 27b is provided in fluid communication with the aerosol generator 26 such that it is capable of receiving generated vapour from the aerosol generator 26. The vapour generation chamber 27b is further provided in fluid communication with air passage 27c. The air passage 27c extends in the region between the reservoir 24 and the outer housing 20a, and passes either side of the reservoir 24, up to the outlet 27d provided at the base of the receptacle 21. Hence, it should be understood that the air pathway in the aerosol provision device 20 extends from the air inlet 27a to the outlet 27d at the base of the receptacle 21, via the vapour generation chamber 27b.

During use of the aerosol provision system, when a user inhales at a mouthpiece end of the aerosol provision system (for example a mouthpiece end of the consumable 30 / proximal end 20b of the housing 20a), air is drawn into the aerosol provision device 20 via the air inlet 27a, passes through the vapour generation chamber 27b where vaporised aerosol-generating material is entrained in the air, before being passed through the air passage 27c and to the air outlet 27d. As will be discussed in more detail below, the aerosol is then passed through the consumable 30 to impart at least one of a flavour and an active substance (e.g., nicotine) to the aerosol before being delivered to the user.

It should be appreciated that the specific form / arrangement of the airflow path is not specific to the principles of the present disclosure, and any suitable arrangement of the airflow path relative to the reservoir 24 may be utilised. For example, the air inlet 27a may be arranged at a different location than that shown in Figure 2, for instance at the distal end 20c of the housing 20a. In other implementations, the reservoir 24 may be an annular chamber having a central opening through which a singular air passage 27c passes, for example.

The aerosol provision device 20 further comprises a reservoir refill mechanism 28. The reservoir refill mechanism 28 is arranged in fluid communication with the reservoir 24 and is configured to allow the reservoir 24 to be refilled with aerosol-generating material, for example from the refill/recharge pack 10. For example, as the aerosol generator 26 is activated, some of the aerosol-generating material in the reservoir 24 is used up. The reservoir refill mechanism 28 is any suitable mechanism that allows the reservoir 24 to be refilled with aerosol-generating material. Refilling the reservoir 24 with aerosol-generating material allows the aerosol provision device 20 to be used multiple times, therefore improving the longevity of the aerosol provision device 20 and reducing material waste. In addition, the reservoir refill mechanism 28 is configured to provide a suitable seal (depending on the nature of the aerosol-generating material) to prevent the escape of aerosol-generating material from the reservoir 24. For example, in some implementations, the reservoir refill mechanism 28 may be valve, such as a spring-loaded ball valve, which is biased into a closed position but capable of being urged to an open position when the reservoir refill mechanism 28 is engaged with a suitable refilling mechanism (e.g., in the refill/recharge pack 10) for refilling of the reservoir 24 with aerosol-generating material. In other implementations, the reservoir refill mechanism 28 may comprise a septum capable of being pierced by a suitable needle or the like of a suitable refilling mechanism (e.g., in the refill/recharge pack 10). The reservoir refilling mechanism 28 is configured to allow aerosol-generating material to pass therethrough.

In the described example, the reservoir refill mechanism 28 is accessible through the receptacle 21. That is, the reservoir refill mechanism 28 is arranged such that the suitable refilling mechanism (e.g., in the refill/recharge pack 10) is capable of passing through an opening in the base of the receptacle 21 and interacting with the reservoir refill mechanism 28. In the present example, the air outlet 27d functions as the opening in the base of the receptacle 21 that allows both the aerosol to pass through the consumable 30 (when installed in the receptacle 21) and the suitable refilling mechanism (e.g., in the refill/recharge pack 10) to engage with the reservoir refill mechanism 28 (when the consumable 30 is not installed in the receptacle 21). However, it should be appreciated that in other implementations, the reservoir refill mechanism 28 may be arranged at an alternate location, for example, on a side of the housing 20a.

Broadly speaking, it should be appreciated that the aerosol provision device 20 of Figure 2 represents an example aerosol provision device 20. The exact arrangement of the components within the housing 20a, such as the aerosol generator 26, reservoir 24, air pathway, etc. may vary from implementation to implementation.

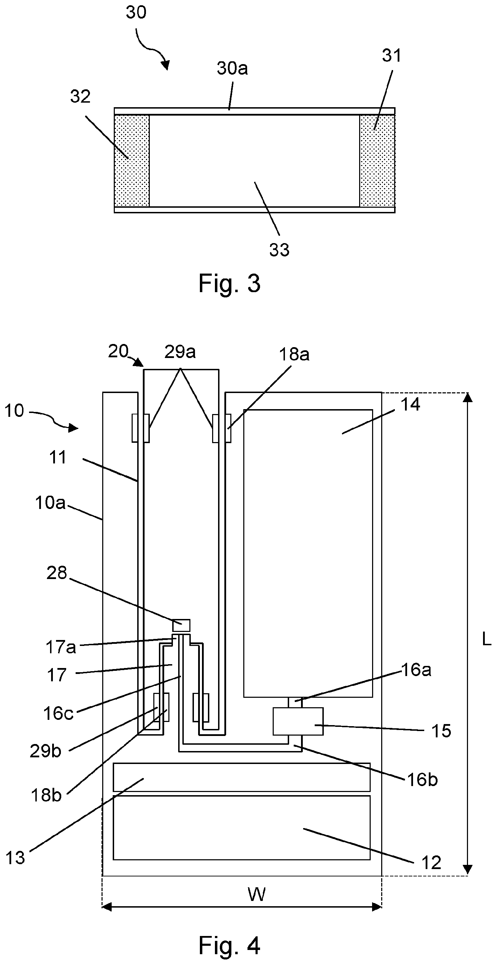

Figure 3 schematically shows the consumable 30 in more detail, in accordance with an aspect of the present disclosure.

The consumable 30 comprises a housing 30a, a first, distal end retaining element 31, a second, proximal end retaining element 32, and an aerosol modifying agent 33.

The housing 30a of the consumable 30 may take any suitable shape, but in the present example is a cylindrical shape. The consumable 30 is sized so as to be received, at least partly, within the receptacle 21 of the aerosol provision device 20, and hence in this example, the receptacle 21 is also similarly cylindrical in shape. The housing 30a may be formed of any suitable material, for example a plastic material. In some implementations, the housing 30a may be formed from paper or a similar material such as card. Forming the housing 30a from paper or card may reduce the manufacturing cost of the consumable 30.

The housing 30a of Figure 3 is shown as being a tubular with openings at either ends thereof. At each end, a retaining element 31, 32 is provided. The retaining elements 31, 32 are positioned to extend across the openings of the tubular housing 30a. Accordingly, the retaining elements 31, 32 and the housing 30a define a volume therebetween. Within the volume is located the aerosol modifying agent 33. Accordingly, it should be understood that the retaining elements 31, 32 help to retain the aerosol modifying agent 33 within the consumable 30. In addition, the retaining elements 31, 32 are configured to allow air (and aerosol from the aerosol provision device 20) to pass through the retaining elements 31, 32. Hence, the retaining elements 31, 32 may take any suitable configuration that allows the retaining elements 31, 32 to both retain the aerosol modifying agent in the volume between the retaining elements 31, 32 and to allow air to pass therethrough. For example, in some implementations, the retaining elements 31, 32 comprise a planar mesh or a porous substrate. In some implementations, the retaining elements 31, 32, and in particular the retaining element 32 at the proximal end of the housing 30a, may be formed from a filter material, for example cellulose acetate. In such implementations, the retaining elements 31, 32 may also act as a filter for filtering certain constituents from the air flow that passes through the retaining elements 31, 32.

The aerosol modifying agent 33 may be any suitable aerosol modifying agent, for example any of the aerosol modifying agents listed above. In broad terms, the aerosol modifying agent 33 is configured to modify one or more properties of the aerosol that passes by or through the aerosol modifying agent 33. In accordance with certain aspects of the disclosure, the aerosol modifying agent 33 comprises an active substance. For example, in one implementation, the aerosol modifying agent 33 comprises nicotine. In accordance with certain aspects of the disclosure, the aerosol modifying agent 33 comprises a flavour or flavourant. For example, in one implementation, the aerosol modifying agent 33 comprises a tobacco flavouring.

In the described implementation, the aerosol modifying agent 33 is or comprises tobacco, for example, cut-rag tobacco, tobacco derivatives, expanded tobacco, reconstituted tobacco, and/or treated tobacco. By using tobacco as the aerosol modifying agent 33, tobacco flavouring and nicotine may be imparted to aerosol passing through the consumable 30 and the aerosol modifying agent 33. In addition, the aerosol that is generated and modified by such a tobacco aerosol modifying agent 33 has similar flavours/tastes to smoke generated by cigarettes, thereby helping to facilitate switching of cigarette users to aerosol provision systems by improving the familiarity of the aerosol provision system to such cigarette users.

With reference to Figures 2 and 3, when the consumable 30 is engaged with the aerosol provision device 20, specifically when the consumable 30 is inserted into receptacle 21, the consumable 30 is inserted such that the distal end (i.e., the end comprising retaining element 31) is inserted first into the receptacle 21. That is, when the consumable 30 is inserted into the receptacle 21, the first, distal retaining element 31 is closest to the distal end 20c of the aerosol provision system 20.

During use of the aerosol provision system (i.e., the combination of the consumable 30 and the aerosol provision device 20), a user places their mouth at the proximal end of the consumable 30 containing the retaining element 32. When the user inhales, air is drawn into the aerosol provision device 20 through the air inlet 27a. In some implementations, the aerosol provision device 20 comprises the pressure sensor 27e (or a similar sensor, such as a flow sensor) which is capable of sensing reduce pressure or air flow through the airflow path as a result of a user inhalation. When the pressure sensor 27e senses air flow (for example, a drop in pressure) resulting from a user inhalation, the control circuitry 23 detects such a drop and subsequently supplies power to the aerosol generator 26 (from battery 22) to cause the aerosol generator 26 to activate, i.e., heat, and generate vapour. In other implementations, the aerosol provision device 20 may comprise a user input mechanism, such as a button (not shown), on the housing 20a of the aerosol provision device 20 which is able to be actuated by a user to cause activation of the aerosol generator 26. When the aerosol generator 26 is activated, aerosol-generating material supplied to the aerosol generator is vaporised / aerosolised and released into the vapour generation chamber 27b. As described above, the vapour is entrained into the airflow and forms an aerosol before being passed along the air passage 27c and out via the outlet 27d. Once the aerosol exits the outlet 27d, the aerosol passes through the retaining element 31 of the consumable 30, through or past the aerosol modifying agent 33, and out of the consumable 30 via the retaining element 32 to be delivered to the user's mouth.

Hence, it should be understood that the aerosol modifying agent 33 acts to modify at least one property of the aerosol that is generated by the aerosol generator 26. In the described implementation, the properties include at least one of: the flavour and the presence of an active substance (such as nicotine). However, it should be appreciated that other properties, for example temperature of the aerosol, may also be affected by the aerosol modifying agent 33.

The consumable 30 is separable from the aerosol provision device 20. In use, when the aerosol modifying agent in the consumable 30 is exhausted (in that it no longer imparts flavour and/or nicotine to the aerosol to an acceptable level), the consumable 30 may be removed from the aerosol provision device 20 and a replacement consumable 30 attached to the device 20 in its place. Provided the reservoir 24 contains sufficient aerosol-generating material, the aerosol provision system can continue to generate aerosol via the aerosol generator 26. In the event that insufficient aerosol-generating material is present in the reservoir 24, the reservoir 24 may be refilled with aerosol-generating material, via the reservoir refill mechanism 28. Therefore, it should be understood that the aerosol provision device 20 is generally regarded as reusable, and usable with multiple (e.g., sequential) consumables 30 which may be regarded as disposable.

The aerosol-generating material may be any suitable aerosol-generating material, as described above. For example, the aerosol-generating material may be a liquid aerosol-generating material, which may be referred to herein as a source liquid, e-liquid or liquid. The source liquid may be broadly conventional, and may contain nicotine and / or other active ingredients, and / or one or more flavours, as described above. In some implementations, the source liquid may contain no nicotine.

In some implementations, the aerosol-generating material stored in the reservoir 24 is free from active ingredients (such as nicotine) and / or flavourants. Without wishing to be bound by theory, when the aerosol-generating material is heated, aerosol-forming material is vaporised / aerosolised and this acts as a transport medium for components such as the active ingredients and / or flavourants which may, in some implementations, not be directly vaporised by the aerosol generator 26 in use. While it is expected that some of the active ingredients and / or flavourants are transported from the aerosol generator 26 by the vaporised aerosol-former material, it has been found that some of the active ingredients / flavourants can be left in contact with the aerosol generator 26. This can lead to residues forming on the aerosol generator 26. These residues can build up on the aerosol generator 26 over time and may subsequently impact the performance of the aerosol generator 26 to provide aerosol to the user. By using an aerosol-generating material that is free from active ingredients (such as nicotine) and/or flavourants, when the aerosol-generating material is aerosolised by the aerosol generator 26 (e.g., via heating), it has been found that a relatively lower amount of residues of the aerosol-generating material are left behind on the aerosol generator 26 (e.g., after vaporisation), therefore prolonging the operational lifetime of the aerosol generator 26. For example, it has been found that in systems that aerosolise an aerosol-generating material that comprises nicotine, for example, the performance of the aerosol generator 26 (e.g., in terms of the mass of aerosol produced for a given puff) starts to decrease after around 5,000 puffs (or discrete activations of the aerosol generator 26). Conversely, in systems that aerosolise an aerosol-generating material that is free of nicotine, for example, the performance of the aerosol generator 26 may not start to decrease until around 20,000 puffs. Accordingly, the lifetime of the aerosol generator 26, and hence of the aerosol provision device 20, may be relatively increased by using such an aerosol-generating material.

In some implementations, the aerosol-generating material is free from any active ingredients and/or flavourants. "Free from" as used herein means that the aerosol-generating material does not contain any active ingredients and/or flavourants or contains no greater than negligible or trace amount of the active ingredients and/or flavourants. More concretely, the aerosol-generating material comprises an active ingredient in an amount of no greater than 0.01 wt.% based on the weight of the aerosol-generating material (such as a liquid aerosol-generating material) and/or the aerosol-generating material comprises one or more flavourants in an amount of no greater than 0.01 wt.% based on the weight of the aerosol-generating material (such as a liquid aerosol-generating material).

In some implementations, the aerosol-generating material comprises, consists of, or essentially consists of an aerosol-former material. As described above, the aerosol-former material may comprise one or more constituents capable of forming an aerosol. In some implementations, the aerosol-former material may comprise one or more of glycerol, propylene glycol, diethylene glycol, triethylene glycol, tetraethylene glycol, 1 ,3-butylene glycol, erythritol, meso-Erythritol, ethyl vanillate, ethyl laurate, a diethyl suberate, triethyl citrate, triacetin, a diacetin mixture, benzyl benzoate, benzyl phenyl acetate, tributyrin, lauryl acetate, lauric acid, myristic acid, and propylene carbonate. In some implementations, the aerosol-generating material may comprise water.

In some implementations, the aerosol-generating material selected from the group comprising, or consisting of: propylene glycol, (vegetable) glycerol, and water. The aerosol-generating material may comprise or consist of any one or combination of the above. In the aerosol provision device 20 of the described implementation, the aerosol generator 26 is provided as an integral part of the aerosol provision device 20. As noted above, the aerosol provision device 20 is intended to be used with multiple consumables 30 and thus the aerosol generator 26 is intended to be used multiple times over a prolonged period of use. In addition, the aerosol generator 26 may be used for a longer period than aerosol provision systems that have a disposable, integrated reservoir and heater (for example, in the form of a disposable cartomiser). Hence, the aerosol generator 26 integrally provided with the aerosol provision device 20 may be more prone to build-up of residue over time which may affect performance if used with an aerosol-generating material having other constituents, such as a flavour or an active substance, for example. Therefore, by using an aerosol-generating material that is free from active ingredients and / or flavourants, the aerosol generator 26 can be used for a longer period of time with relatively lower levels of residual build-up, thereby maintaining acceptable performance for longer.

However, in such implementations, while use of the aerosol-generating material that is free from active ingredients and / or flavourants has certain advantages, the aerosol generated therefrom is typically unflavoured and does not contain an active substance (such as nicotine). Particularly for consumers who are transitioning from cigarettes or the like to aerosol provision systems, the presence of flavour and/or active substance may be qualities in an aerosol that these consumers are looking to be provided with, and the absence of these qualities may lead to such users reverting back to cigarettes or the like. Therefore, as described above, the consumable 30 comprises an aerosol modifying agent 33, which is positioned along the airflow path (e.g., at the air outlet 27d of the aerosol provision device 20) such that the aerosol generated by the aerosol generator 26 passes to or through the aerosol modifying agent 33 to modify a characteristic of the aerosol. In the described implementation, the aerosol modifying agent 33 is capable of imparting a (tobacco) flavour and/or active substance (nicotine) to the aerosol generated by the aerosol generator 26 as the aerosol passes to or through the aerosol modifying agent 33 (which, as noted above, may be or comprise a tobacco or tobacco based substance). In this way, despite the aerosol-generating material not containing any flavour (at least beyond any flavour of the propylene glycol / (vegetable) glycerol) and/or active substance, the aerosol modifying agent 33 is capable of providing flavour and/or an active substance to the aerosol that is delivered to the user.

It should be appreciated, however, that in other implementations the aerosol-generating material may contain an active substance and/or flavour. In such implementations, the consumable 30 may be configured to modify additional or alternative characteristics of the aerosol generated from the aerosol-generating material. For example, the consumable 30 may impart an additional flavour, and/or reduce the temperature of the aerosol, and/or impart an additional active substance.

In the above example, the receptacle 21 provides a location where the consumable 30 couples or otherwise engages with the aerosol provision device 20. In the above example, the consumable 30 is held in place in the receptacle 21 by friction-fit. That is, the diameter of the receptacle 21 may be the same size as (or slightly smaller than, e.g., 0.1 or less of a mm) the diameter of the consumable 30. However, in other implementations, the consumable 30 may be engaged with the receptacle 21 in other ways, for example based around a screw thread, latch mechanism, bayonet fixing or magnetic coupling. In addition, it should be appreciated that the receptacle 21 represents only an example of a suitable interface for interfacing with and engaging the consumable 30. In other implementations, the consumable 30 may be engaged with the aerosol provision device 20 in any suitable way.

In the described example, in use, the user places their mouth on the outer housing 30a of the consumable 30 (at the proximal end thereof). However, it should be appreciated that in some implementations, the consumable 30 may be completely contained within the aerosol provision device 20. Accordingly, a part of the housing 20a (for example, a removable cover that provides access to the receptacle 21) may alternatively form the mouthpiece for the aerosol provision system.

In other implementations, the consumable 30 may be omitted and instead the aerosol provision device 20 is provided with a mouthpiece through which the aerosol generated by the aerosol generator 26 from the aerosol-generating material in the reservoir 24 is capable of being delivered to a user.

Figure 4 schematically shows the refill/recharge pack 10 in more detail, along with a schematic representation of the aerosol provision device 20, in accordance with an aspect of the present disclosure. It should be appreciated that Figure 4 is not shown to any particular scale and the various components are only schematically shown. In addition, it should be appreciated that certain features of the refill/recharge pack 10 are omitted from Figure 4, such as the various wiring and electrical connections between certain components, for example.

The refill/recharge pack 10 comprises a housing 10a, receptacle 11 for receiving the aerosol provision device 20, power source 12, control circuitry 13, reservoir 14, transfer mechanism 15, aerosol-generating material conduit 16a, 16b and 16c, aerosol provision device engagement mechanism 17, and electrical contacts 18a, 18b.

The refill/recharge pack 10 is configured to have the shape and dimensions of a cigarette pack. That is, the refill/recharge pack 10 may be broadly cuboidal and have a dimension in the length direction L (i.e., along a longitudinal axis thereof) of between 14 cm and 6 cm, or between 13 cm and 7 cm, or between 10 cm and 8 cm, a total width dimension W (i.e., perpendicular to the longitudinal axis) of between 8 cm and 5 cm, or between 7 cm and 5.5 cm, and a total depth dimension (not shown in Figure 4, but perpendicular to both the longitudinal axis and width direction) of between 3 cm and 1 cm, or between 2.5 cm and 1.5 cm. The dimension in the length direction L may be dependent on the length of the aerosol provision device 20.

In the example of Figure 4, the refill/recharge pack 10 comprises the receptacle 11 which is sized so as to receive the aerosol provision device 20. For example, the receptacle 11 may define a cylindrical recess having a similar width / diameter and length as the aerosol provision device 20. The receptacle 11 has a longitudinal axis that is parallel with the length dimension of the refill/recharge pack 10. In Figure 4, the distal end of the aerosol provision device 20 protrudes out of the receptacle 11 when the aerosol provision device 20 is fully inserted. This may aid in allowing a user to remove the aerosol provision device 20 from the refill/recharge pack 10 by providing a region of the aerosol provision device 20 for the user to grip (e.g., with their thumb and forefinger). In some implementations, the aerosol provision device 20 may be fully enclosed within the receptacle 11. In such implementations, a mechanism may be implemented to help lift the aerosol provision device 20 from the receptacle 11 for removal from the refill/recharge pack 10.

The refill/recharge pack 10 is intended to receive the aerosol provision device 20 between uses of the aerosol provision device 20 / system, to recharge the battery 22 of the aerosol provision device 20 and to refill the reservoir 24 of the aerosol provision device 20. When the user removes the aerosol provision device 20 from the refill/recharge pack 10 after recharging and refilling is complete, the action is similar to removing a cigarette from a cigarette packet. In this regard, providing the refill/recharge pack 10 with similar dimensions to a cigarette pack increases familiarity to users transitioning from cigarettes to electronic aerosol provision systems, which therefore may help ease such a transition. In some implementations, the refill/recharge pack 10 may also have a similar weight to a cigarette pack, for broadly similar reasons.

The outer housing 10a in the described implementation has an overall cuboidal shape having a top surface (through which the receptacle 11 is accessible), a bottom surface opposite the top surface, and one or more side surfaces extending therebetween and perpendicular thereto. The outer housing 10a may be formed, for example, from a plastics or metallic material. In some implementations, the outer housing 10a may be circumscribed, at least partly, by a paper material or cellulose material. Within the outer housing 10a is located the various components of the refill/recharge pack 10, such as the power source 12, control circuitry 13, etc.

The power source 12 in this implementation is a battery 12. The battery 12 may be rechargeable, for example a lithium ion battery, although other battery chemistries may also be considered. The battery 12 is capable of being recharged via an external source (such as via a connection to mains power through a suitable cable, not shown, or via inductive charging). In some implementations, the battery 12 may not be rechargeable. In such implementations, the outer housing 10a may comprise a door or hatch that allows for the battery 12 to be replaced with a fresh (i.e., charged) battery 12. The battery 12 is intended to be used to recharge the battery 22 of the aerosol provision device 20, and thus has a capacity at least equal to the capacity of the battery 22 of the aerosol provision device 20. However, in some implementations, the capacity of the battery 12 of the refill/recharge pack 10 may be greater, for example 5 or more, 10 or more, or 20 or more times greater than the capacity of the battery 22 of the aerosol provision device 20. This means that the refill/recharge pack 10 is capable of recharging the battery 22 of the aerosol provision device 20 multiple times on a single charge.

The control circuitry 13 is suitably configured / programmed to control the operations of the refill/recharge pack 10. The control circuitry 13 may be considered to logically comprise various sub-units / circuitry elements associated with different aspects of the refill/recharge pack's operation and may be implemented by provision of a (micro)controller, processor, ASIC or similar form of control chip. The control circuitry 13 may be arranged to control any functionality associated with the refill/recharge pack 10. By way of non-limiting examples only, the functionality may include the charging or re-charging of the battery 12 (e.g., from the external source), the discharging of the battery 12 (e.g., for recharging the battery 22 of the aerosol provision device 20), and the transfer of aerosol generating material from the reservoir 14 to the reservoir 24 of the aerosol provision device 20. In some examples, other functionality such as controlling visual indicators (e.g., LEDs) / displays of the refill/recharge pack 10, communication functionality for communicating with external devices, etc. may also be controlled by the control circuitry 13. The control circuitry 13 may be mounted to a printed circuit board (PCB). Note also that the functionality provided by the control circuitry 13 may be split across multiple circuit boards and / or across components which are not mounted to a PCB, and these additional components and / or PCBs can be located as appropriate within the refill/recharge pack 10.

The refill/recharge pack 10 further comprises a reservoir 14 configured to store aerosol-generating material. In the present example, the reservoir 14 is configured to store a liquid aerosol-generating material, and may therefore be configured so as to reduce or prevent leakage of the aerosol-generating material out of the reservoir 14. The reservoir 14 may take any suitable shape, such as a cuboidal shape.

The aerosol-generating material in the reservoir 14 is intended to be transferred to the reservoir 24 of the aerosol provision device 20 when the aerosol provision device 20 is installed in the receptacle 11. The reservoir 14 is intended to be used to refill the reservoir 24 of the aerosol provision device 20, and thus in some implementations has a volume at least equal to the volume of the reservoir 24 of the aerosol provision device 20. However, in some implementations, the volume of the reservoir 12 of the refill/recharge pack 10 may be greater, for example 5 or more, 10 or more, or 20 or more times greater than the volume of the reservoir 24 of the aerosol provision device 20. This means that the refill/recharge pack 10 is capable of refilling the reservoir 24 of the aerosol provision device 20 multiple times from the reservoir 12 of the refill/recharge pack 10.

In some implementations, the reservoir 14 may be integrally formed with the refill/recharge pack 10, and may or may not be refillable. However, in other implementations, the reservoir 14 may be removable from the refill/recharge pack 10. In these implementations, once the reservoir 14 is depleted, the reservoir 14 may be removed and replaced with a new (full) reservoir 14.

In the present implementation, at the base of the receptacle 11 of the refill/recharge pack 10, an aerosol provision device engagement mechanism 17 for engaging with the aerosol provision device 20 is provided. The engagement mechanism 17 is sized so as to be received in the receptacle 21 of the aerosol provision device 20. That is, the aerosol provision device 20 is inserted into the receptacle 11, proximal end 20b first such that as the aerosol provision device 20 is lowered into the receptacle 11, the engagement mechanism 17 aligns with and engages the receptacle 21 of the aerosol provision device 20.

As described previously, the reservoir refill mechanism 28 is accessible through the base of the receptacle 21. The engagement mechanism 17 is configured to engage with the reservoir refill mechanism 28 so as to facilitate refilling of the reservoir 24 of the aerosol provision device 20. In particular, the engagement mechanism 17 comprises a protrusion 17a at an end thereof that is arranged to protrude through the outlet 27d of the aerosol provision device 20 and engage with, and actuate (e.g., move the spring-loaded ball valve of) the reservoir refill mechanism 28. The protrusion 17a may be suitably shaped to engage with the reservoir refill mechanism 28, and may take different forms in dependence on the specific reservoir refill mechanism 28 employed in the aerosol provision device 20. For example, if the reservoir refill mechanism 28 is a septum, the protrusion 17a may take the form of a needle.

The refill/recharge pack 10 comprises an aerosol-generating material conduit formed of three parts; a first conduit 16a in fluid communication with the reservoir 14 and extending to an input of the transfer mechanism 15, a second conduit 16b in fluid communication with the output of the transfer mechanism 15 and extending to and in fluid communication with a third conduit 16c extending through the engagement mechanism 17 and the protrusion 17a and terminating at an opening at the end of the protrusion 17a. Together, the first, second and third conduits16a, 16b, 16c are referred to herein as aerosol-generating material conduit 16. The aerosol-generating material conduit 16 (or conduit 16) is configured to allow aerosol-generating material from the reservoir 14 to pass along the conduit 16 and to the reservoir 24 of the aerosol provision device 20 via the reservoir refill mechanism 28. That is, the conduit 16 is capable of supplying aerosol-generating material to an opening in the protrusion 17a, which when the aerosol provision device 20 is installed in the receptacle 11, supplies aerosol-generating material to the reservoir 24 to refill the reservoir 24 of the aerosol provision device 20. In the present example, the conduit 16 is configured to transport liquid aerosol-generating material to the opening in the protrusion 17a.

It should be appreciated that aerosol provision device 20 and the refill/recharge pack 10 each comprise a respective interface at which the two engage when the aerosol provision device 20 is inserted into the receptacle 11 of the refill/recharge pack 10. The respective interfaces include at least the components that are considered to be brought into contact with one another and are exposed to aerosol-generating material during use. For example, the refill/recharge pack 10 may be said to comprise an aerosol provision device interface, which may include the protrusion 17a and/or the opening of the conduit 16c. The aerosol provision device 20 may be said to comprise a refill/recharge pack interface, which includes at least a part of the reservoir refill mechanism 28. Each of these components as described above are understood to come into direct contact with the aerosol-generating material at regions of the aerosol provision device 20 and refill/recharge pack 10 that are exposed to the outside environment. For example, aerosol-generating material flows through conduit 16c at the tip of the protrusion 17a and may also contact the surface of the protrusion 17a. Equally, the aerosol-generating material flows through the reservoir refill mechanism 28 and subsequently contacts surfaces of the reservoir refill mechanism 28 as it passes to the reservoir 24.

The respective interfaces (i.e., the aerosol provision device interface of the refill/recharge pack 10 and the refill/recharge pack interface of the aerosol provision device 20) may not extend to just the regions of the respective components that directly receive aerosol-generating material, but may extend to the surrounding areas of the respective components that are capable of receiving, either directly or indirectly, aerosol-generating material. For example, the aerosol provision device interface of the refill/recharge pack 10 may additionally include the outer surface of the engagement mechanism 17, while the refill/recharge pack interface of the aerosol provision device 20 may include the inner surface of the receptacle 21. These areas may not be in direct contact with aerosol-generating material, at least during normal use, but may nonetheless be exposed to aerosol-generating material if, for example, any material drips or leaks from the reservoir refill mechanism 28 or the protrusion 17a or conduit 16c as the aerosol provision device 20 is removed from the receptacle 11 after a refilling procedure has been performed.

The arrangement in Figure 4 depicts an example arrangement of the aerosol provision device 20 and the refill/recharge pack 10, and the aerosol provision device interface of the refill/recharge pack 10 and the refill/recharge pack interface of the aerosol provision device 20 are defined in a particular manner. However, it should be appreciated that the aerosol provision device interface of the refill/recharge pack 10 and the refill/recharge pack interface of the aerosol provision device 20 may be defined in any suitable manner for a given system 1. More generally, the aerosol provision device interface of the refill/recharge pack 10 and the refill/recharge pack interface of the aerosol provision device 20 include those areas / interfaces of aerosol provision device 20 and the refill/recharge pack 10 that are capable of receiving or being brought into contact with the aerosol-generating material. The refill/recharge pack 10 further comprises a transfer mechanism 15, which in this implementation is in fluid communication with the conduit 16. The transfer mechanism 15 may be any mechanism suitable of driving the transfer of aerosol-generating material from the reservoir 14 along the conduit 16 and to the reservoir 24 of the aerosol provision device 20.

Any suitable transfer mechanism 15 may be used in accordance with the principles of the present disclosure. In the described implementation, the transfer mechanism 15 is a peristaltic pump. A peristaltic pump typically works through rotary motion, where a rotatable component comprises a number of rollers or the like that act to compress a flexible tube as they rotate, forcing the fluid to move through the tube. The conduit 16 is provided either side of the transfer mechanism 15 (i.e., coupling to an inlet and an outlet of the transfer mechanism 15). However, other pumps may be used in place of the peristaltic pump. In addition, the transfer mechanism 15 may alternatively include other mechanisms, such as a plunger or the like in the reservoir 14 and arranged to change the volume of the reservoir 14 to force aerosol-generating material along the conduit 16. In this case, it should be appreciated that the transfer mechanism 15 is not located on the conduit 16. The transfer mechanism 15 may be provided at a suitable location in the refill/recharge pack 10.

In some implementations, the transfer mechanism 15 may be electronically operated. For example, a motor provided in the refill/recharge pack 10, under control of the control circuitry 13, may be used to drive the peristaltic pump or other transfer mechanism 15. The battery 12 may be used to supply power to the transfer mechanism 15 in such implementations. Control of the motor may be dependent on a suitable detection process for detecting that the aerosol provision device 20 is located in the receptacle 11; that is, the transfer mechanism 15 may be operable to transfer aerosol-generating material from the reservoir 14 when the aerosol provision device 20 is determined to be present in the receptacle 11. Alternatively, the transfer mechanism 15 may be mechanically and manually operated by a user; for example, by a rotatable arm coupled to the rotational axis of the peristaltic pump and provided on the outer surface of the housing 10a.

In some implementations, the transfer mechanism 15 may be omitted.

Hence, in general, the refill/recharge pack 10 is configured to cause aerosol-generating material provided in the reservoir 14 to pass to the reservoir 24 of the aerosol provision device 20 when the aerosol provision device 20 is located in the receptacle 11. Accordingly, the reservoir 24 of the aerosol provision device 20 is capable of being refilled with aerosol-generating material by the refill/recharge pack 10.

In addition, the refill/recharge pack 10 comprises electrical contacts 18a and 18b. The electrical contacts 18a, 18b are arranged in the refill/recharge pack 10 such that they are able to be brought into electrical connection with the electrical contacts 29a, 29b of the aerosol provision device 20 when the aerosol provision device 20 is located in the receptacle 11. More specifically, a first electrical contact 18a of the refill/recharge pack 10 is arranged so as to electrically connect with the first electrical contact 29a of the aerosol provision device 20, and a second electrical contact 18b is arranged so as to electrically connect with the second electrical contact 29b of the aerosol provision device 20.

In Figure 4, the first electrical contact 18a of the refill/recharge pack 10 is provided at a location toward the opening of the receptacle 11 such that when the aerosol provision device 20 is inserted into the receptacle 11, the first electrical contact 29a at the distal end 20c of the aerosol provision device 20 is capable of being bought into electrical connection with the first electrical contact 18a of the refill/recharge pack 10. The first electrical contact 18a may take any suitable form; for example, the electrical contact 18a may be an annular ring provided extending, coaxially, with the axis of the receptacle 11. In other implementations, the electrical contact 18a may comprise one or more contact pads provided at a surface of the receptacle 11. In some implementations, the radial extent of the first electrical contact 29a of the aerosol provision device 20 about the longitudinal axis of the aerosol provision 20 and the radial extent of the first electrical contact 18a of the refill/recharge pack 10 about the longitudinal axis of the receptacle 11 is such that the aerosol provision device 20 can be inserted at any rotational position about the longitudinal axis of the aerosol provision device 20 relative to the receptacle 11 and still provide electrical contact between the first electrical contacts 18a, 29a. For example, the electrical contact 29a may extend 360° around the longitudinal axis of the aerosol provision device 20, and the electrical contact 18a may extend between 1° to 360° around the longitudinal axis of the receptacle 11.

In Figure 4, the second electrical contact 18b of the refill/recharge pack 10 is provided at a location on the outer surface of the engagement mechanism 17. In this case, when the aerosol provision device 20 is inserted into the receptacle 11, the second electrical contact 29b located within the receptacle 21 of the aerosol provision device 20 is capable of being bought into electrical connection with the second electrical contact 18b of the refill/recharge pack 10 when the engagement mechanism 17 engages with the receptacle 21. The second electrical contact 18b may take any suitable form; for example, the electrical contact 18b may be an annular ring provided extending, coaxially, with the axis of the engagement mechanism 17. In other implementations, the electrical contact 18b may comprise one or more contact pads provided at a surface of the engagement mechanism 17. In some implementations, the radial extent of the second electrical contact 29b of the aerosol provision device 20 about the longitudinal axis of the receptacle 21 and the radial extent of the second electrical contact 18a of the refill/recharge pack 10 about the longitudinal axis of the engagement mechanism 17 is such that the aerosol provision device 20 can be inserted at any rotational position about the longitudinal axis of the aerosol provision device 20 relative to the receptacle 11 and still provide electrical contact between the second electrical contacts 18b, 29b. For example, the electrical contact 29b may extend 360° around the longitudinal axis of the receptacle 21, and the electrical contact 18b may extend between 1° to 360° around the longitudinal axis of the engagement mechanism 17.

When the aerosol provision device 20 is installed in the receptacle 11 of the refill/recharge pack 10, the refill/recharge pack 10 is configured to recharge the battery 22 of the aerosol provision device 20. The battery 12 of the refill/recharge pack 10 is electrically connected (potentially via recharging circuitry of the control circuitry 13) to the first electrical contact 18a and the second electrical contact 18b. When the aerosol provision device 20 is inserted into the receptacle 11, and the first electrical contact 29a of the aerosol provision device 20 bought into electrical connection with the first electrical contact 18a of the refill/recharge pack 10 and the second electrical contact 29b of the aerosol provision device 20 bought into electrical connection with the second electrical contact 18b of the refill/recharge pack 10, the refill/recharge pack 10 is configured to cause recharging of the battery 22 of the aerosol provision device 20 by applying suitable power from the battery 12 of the refill/recharge pack 10. Although not shown in Figure 4, the refill/recharge pack 10 and/or the aerosol provision device 20 may have suitable circuitry to control and/or monitor the recharging of the battery 24 of the aerosol provision device 20, for example to help ensure the recharging is performed safely and accurately.

Hence, in this example, the refill/recharge pack 10 is also configured to cause electrical power provided in the battery 12 of the refill/recharge pack 10 to pass to the battery 22 of the aerosol provision device 20 when the aerosol provision device 20 is located in the receptacle 11. Accordingly, the battery 22 of the aerosol provision device 20 is capable of being recharged with electrical power by the refill/recharge pack 10.

In accordance with the principles of the present disclosure, a sterilisation component is provided to one or both of the aerosol provision device 20 and the refill/recharge pack 10. As described above, the aerosol provision device 20 includes a refill/recharge pack interface and the refill/recharge pack 10 includes an aerosol provision device interface which are interfaces of the respective components that are bought into engagement with one another during a refilling operation and across which aerosol-generating material is intended to pass. The sterilisation component is arranged so as to prevent or reduce microbial growth at at least one of the interfaces (i.e., the refill/recharge pack interface of the aerosol provision device 20 and the aerosol provision device interface of the refill/recharge pack 10).

As should be appreciated from Figure 4, when the aerosol provision device 20 and the refill/recharge pack 10 are coupled, the refill/recharge pack interface of the aerosol provision device 20 and the aerosol provision device interface of the refill/recharge pack 10 are coupled in such a way so as to allow aerosol-generating material to pass across, at least a part of, the coupled interfaces (e.g., from the aerosol provision device interface of the refill/recharge pack 10 via the protrusion 17a or opening of conduit 16c to the refill/recharge pack interface of the aerosol provision device 20 via the reservoir refill mechanism 28). Accordingly, during use, aerosol-generating material contacts these components of the respective interfaces, and potentially remains on or at the interfaces even after the aerosol provision device 20 has been removed from (decoupled from) the refill/recharge pack 10. As discussed above, there may even be transport of the aerosol-generating material to other regions of the refill/recharge pack interface of the aerosol provision device 20 and the aerosol provision device interface of the refill/recharge pack 10, which may occur for example due to dripping and/or leaking of the aerosol-generating material from the protrusion 17a / conduit 16c or the reservoir refill mechanism 28.

Aerosol-generating material that remains at the refill/recharge pack interface of the aerosol provision device 20 and/or the aerosol provision device interface of the refill/recharge pack 10 may be prone to microbial growth, particularly when exposed to the environment. To some extent, the composition of the aerosol-generating material may be modified to help reduce the extent of microbial growth. For example, nicotine has been shown to inhibit microbial growth, and therefore aerosol-generating materials that comprise nicotine may display relatively lower levels of microbial growth over time compared to aerosol-generating materials that do not contain nicotine. It should be appreciated that there may be other constituents that inhibit microbial growth that may be additionally or alternatively incorporated into the aerosol-generating material.

In some implementations, the aerosol-generating material is free from active ingredients and / or flavourants. Such aerosol-generating material may be particularly prone to microbial growth and, in some instances, may even provide a particularly effective environment for microbial growth.

Hence, by use of a sterilisation component, as will be described in more detail below, microbial growth can be inhibited (or further inhibited, e.g., if the aerosol-generating material comprises a substance such as nicotine with antimicrobial properties), thereby keeping the refill/recharge pack interface of the aerosol provision device 20 and/or the aerosol provision device interface of the refill/recharge pack 10 relatively free of microbes. Accordingly, the refill/recharge pack 10 and/or aerosol provision device 20 may be considered relatively more hygienic to users despite the presence of the refill/recharge pack interface of the aerosol provision device 20 and/or the aerosol provision device interface of the refill/recharge pack 10 used for refilling the reservoir 24 of the aerosol provision device 20 which are, at least in some instances, exposed to the environment.

As noted above, the sterilisation component is arranged to reduce or prevent microbial growth at the refill/recharge pack interface of the aerosol provision device 20 and/or the aerosol provision device interface of the refill/recharge pack 10. The sterilisation component may take any suitable form. In addition, the sterilisation component may be an active sterilisation component, in that the sterilisation component is selectively controlled or powered to provide a sterilisation effect, or the sterilisation component may be a passive sterilisation component, in that the sterilisation component continuously provides a sterilisation effect without any control and/or power.

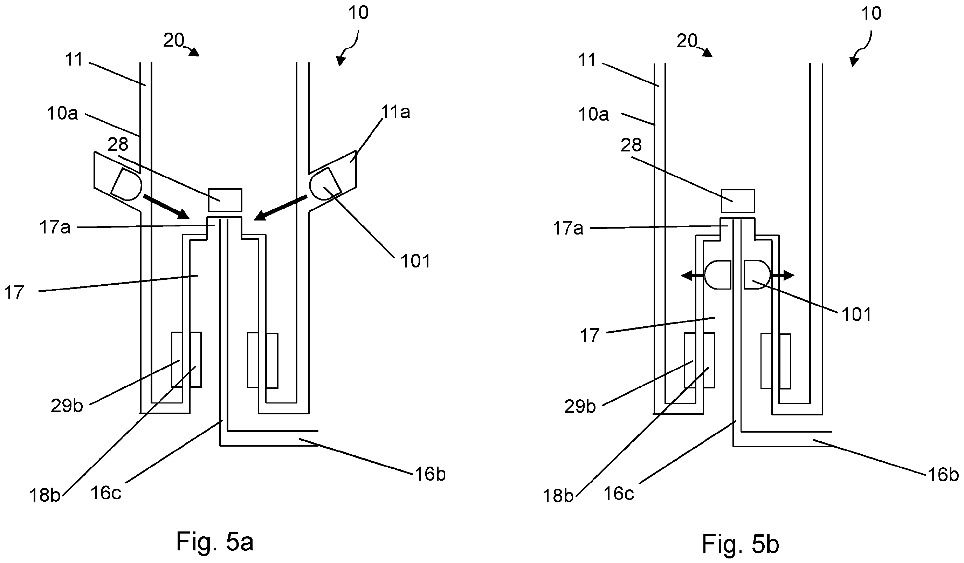

Figures 5a and 5b schematically represent two implementations of an active sterilisation component provided as part of the refill/recharge pack 10. Figures 5a and 5b show, in more detail, a part of the receptacle 11 of the refill/recharge pack 10 and the aerosol provision device 20 installed in the receptacle 11 of Figure 4. In particular, Figures 5a and 5b show the lower half of the receptacle 11 including the engagement mechanism 17 and the second and third conduits 16b and 16c. It will be appreciated that certain features of Figure 4 have been omitted for clarity (such as the control circuitry 13, reservoir 14, etc.).

In both of Figures 5a and 5b, the sterilisation component comprises one or more UV emitting lights 101. In Figures 5a and 5b a pair of UV emitting lights 101 is shown, however it should be appreciated that in other implementations only a single UV emitting light 101 or more than two UV emitting lights 101 may be provided.

The UV emitting lights 101 are arranged to receive electrical power from a power source, such as the battery 12 of the refill/recharge pack 10, to generate and output UV light. UV, or ultraviolet, light is typically light or electromagnetic radiation having a wavelength of between 400 nm to 10 nm. The UV emitting lights 101 of the present implementations may be configured to output UV light having a wavelength within the range 10 nm to 400 nm. In some implementations, the UV emitting lights 101 are configured to output light at a wavelength of between 100 to 280 nm (so-called UV-C, or shortwave ultraviolet). It has been found that shortwave ultraviolet light is particularly effective at reducing or preventing microbial growth, although it should be appreciated ultraviolet light at other wavelengths may also display some anti-microbial properties. The activation of the UV emitting lights 101 in this implementation is controlled by the control circuitry 13. Thus, the control circuitry 13 is configured to control the power supply from the battery 12 to the UV emitting lights 101 to cause the UV emitting lights 101 to output suitable UV light. The UV emitting lights 101 may be any form of suitable light source capable of emitting suitable UV light, such as UV lamps, LEDs, lasers, etc.

Turning to Figure 5a, the UV emitting lights 101 are located in corresponding wells or recesses 11a provided in the walls of the receptacle 11. That is, the recesses 11a are recessed from the wall of the receptacle 11. In this implementation, the recesses 11a extend into the refill/recharge pack 10 from the receptacle 11 along a direction of extent, and that direction of extent is arranged at an inclined angle relative to the longitudinal axis of the receptacle 11. That is, for example, the recesses 11a may generally define a direction of extent (which aligns with the arrows shown in Figure 5a), and this direction of extent is provided at an angle of approximately 60° to 70° relative to the longitudinal axis of the receptacle 11.

The UV emitting lights 101 are arranged in the recesses 11a and are corresponding arranged to emit UV light predominantly along the direction of extent of the recesses 11a into the receptacle 11. More particularly, the UV light is directed towards the protrusion 17a of the engagement mechanism 17 and the opening of the conduit 16c. In other words, the UV light is predominately directed towards the aerosol provision device interface of the refill/recharge pack 10. This is schematically shown by the arrows in Figure 5a. As should be appreciated, by virtue of the inclined recesses 11a in which the UV emitting light 101 is housed, the direction of the UV light emitted by the UV emitting light 101 is similarly angled (or inclined) with respect to the longitudinal axis.

The configuration shown in Figure 5a directs the UV light broadly in a direction towards the base of the receptacle 11 (that is, away from the open end of the receptacle 11). In some implementations, this may prevent or reduce the amount of UV light escaping the receptacle 11. However, this need not be the case in each and every implementation, and the recesses 11a and/or the direction of the UV light need not be inclined to the longitudinal axis of the receptacle (e.g., it may instead be arranged at 90° to the receptacle). Additionally, it should be appreciated that the angle of incline of the recesses 11a relative to the longitudinal axis of the receptacle 11 may depend on the position of the recesses along the longitudinal axis of the receptacle 11 and/or the intended direction along which UV light is to be predominately emitted from the UV emitting light 101. In some implementations, it should also be appreciated that for two or more UV emitting lights 101, the angle of incline of the recesses 11a and/or the direction of UV light emitted from the UV emitting lights 101 may be different from one another. This may help to, in effect, increase the area of irradiation of UV light on the aerosol provision device interface.

In the context of Figure 5a, the UV emitting lights 101 are controlled to activate (i.e., emit UV light) when the aerosol provision device 20 is not located in the receptacle 11. Therefore, as hinted at above, the UV light emitted from the UV emitting lights 101 is arranged to illuminate / irradiate the aerosol provision device interface, and more particularly the protrusion 17a and the opening of the conduit 16. Therefore, the interface and/or any aerosol-generating material disposed thereon, is exposed to UV light from the UV emitting lights 101, thereby inhibiting or preventing microbial growth on these surfaces.

The UV emitting lights 101 may be controlled to activate upon detection of the aerosol provision device 20 being removed from the receptacle 11. Alternatively, or additionally, the UV emitting lights 101 may be controlled to periodically activate. In some implementations, the UV emitting lights 101 may be periodically activated regardless of whether the aerosol provision device 20 is in the receptacle 11 or not. However, to save battery power, in other implementations, the UV emitting lights 101 may be periodically activated provided the aerosol provision device 20 is determined as not being present in the receptacle 11 (e.g., via output from a suitable sensor provided in the receptacle 11), where periodic activation of the UV emitting lights 101 may be suspended when the aerosol provision device 20 is determined as being located in the receptacle 11.

Turning to Figure 5b, the UV emitting lights 101 in this implementation are located in the engagement mechanism 17 of the refill/recharge pack 10. In particular, in this implementation, the UV emitting lights 101 are located in recesses (not shown) in the engagement mechanism 17 and are broadly arranged to emit UV light in a direction that is 90° to the longitudinal axis of the receptacle 11. This is shown by the arrows in Figure 5b.

The UV emitting lights 101 of Figure 5b are the same as those in Figure 5a. However, in this implementation, the UV emitting lights 101 are arranged to emit UV light in a directed towards the receptacle 21 of the aerosol provision device 20. In other words, the UV light is predominately directed towards a part of the refill/recharge pack interface of the aerosol provision device 20. This is schematically shown by the arrows in Figure 5b.

In the context of Figure 5b, the UV emitting lights 101 may be controlled to activate (i.e., emit UV light) when the aerosol provision device 20 is located in the receptacle 11. For example, a suitable sensor may be located in the receptacle 11 to detect whether the aerosol provision device 20 is located in the receptacle 11 or not, and when it is determined that the aerosol provision device 20 is located in the receptacle 11, the control circuitry 13 is configured to cause UV emitting lights 101 to activate. When the aerosol provision device 20 is present in the receptacle 11, the UV light emitted from the UV emitting lights 101 is arranged to illuminate / irradiate the refill/recharge pack interface, and more particularly the receptacle 21. Therefore, the interface and/or any aerosol-generating material disposed thereon, is exposed to UV light from the UV emitting lights 101, thereby inhibiting or preventing microbial growth on these surfaces. The UV emitting lights 101 may be controlled to activate while refilling of the reservoir 24 is being performed, or additionally or alternatively, the UV emitting lights 101 may be controlled to emit UV light periodically when the aerosol provision device 20 is located in the receptacle 11 (e.g., when the aerosol provision device 20 is stored in the refill/recharge pack between uses / refilling operations).

It should be appreciated that the position of the UV emitting lights 101 in Figure 5b is provided as an example, and the UV emitting lights 101 may be arranged to emit UV light in any suitable direction. For example, the UV emitting lights 101 may additionally or alternatively be arranged to emit light in a direction along the longitudinal axis of the receptacle 11 (e.g., upwards from surface of the engagement mechanism 17). Although the UV emitting lights 101 are shown as emitting UV light in a direction of 90° to the longitudinal axis of the receptacle 11, it should be appreciated that the UV emitting lights 101 may be arranged to emit the UV light in a direction that is inclined relative to the longitudinal axis of the receptacle 11.

In both implementations described by Figures 5a and 5b, the UV emitting lights 101 are controlled to activate for a period of time that is sufficient to prevent or reduce microbial growth. The precise duration may be determined empirically or through computer modelling. The period of time may depend on the aerosol-generating material that is used to refill the reservoir 24 of the aerosol provision device 20. The period of time may alternatively or additionally depend on the properties of the UV emitting lights 101 and/or the number of UV emitting lights 101.

In the described implementations of Figures 5a and 5b, the UV emitting lights 101 are provided as part of the refill/recharge pack 10, and are correspondingly controlled by the control circuitry 13 of the refill/recharge pack 10 and powered by the power source 12 of the refill/recharge pack 10. However, in other implementations, the UV emitting lights 101 may be formed as part of the aerosol provision device 20.

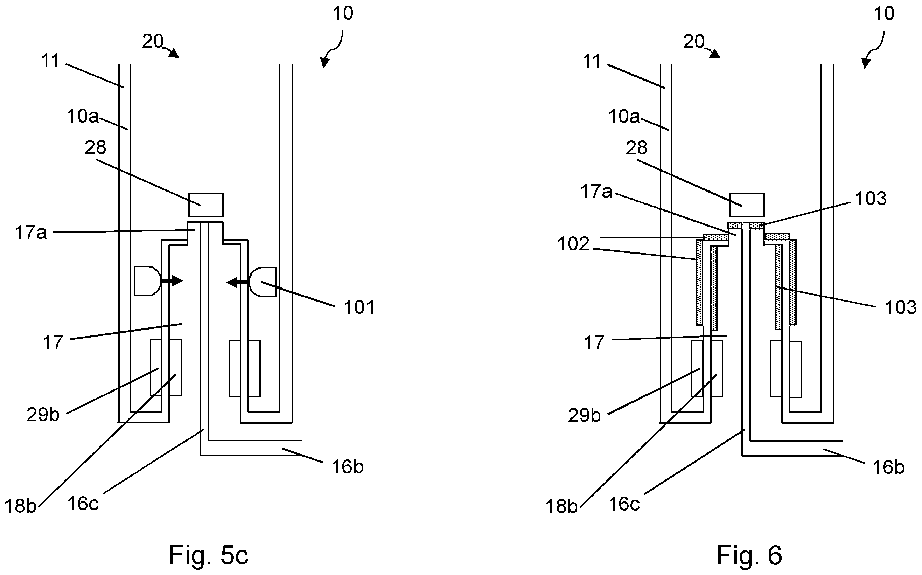

Figure 5c schematically represent a further implementation of an active sterilisation component comprising UV emitting lights 101 provided, in this implementations, as part of the aerosol provision device 20. As with Figures 5a and 5b, Figure 5c shows the lower half of the receptacle 11 including the engagement mechanism 17 and the second and third conduits 16b and 16c. It will be appreciated that certain features of Figure 4 have been omitted from Figure 5c for clarity (such as the control circuitry 13, reservoir 14, etc.).

In Figure 5c, a pair of UV emitting lights 101 (which are the same as the UV emitting lights 101 of Figures 5a and 5b) are provided in the aerosol provision device 20. In particular, the UV emitting lights 101 are provided at the proximal end 20b of the aerosol provision device 20 and are orientated such that the light emitted from the UV emitting lights 101 is predominantly directed into the receptacle 21. Suitable recesses (not shown) in the walls of the receptacle 21 may be provided for accommodating the UV emitting lights 101.

When the aerosol provision device 20 is engaged with the refill/recharge pack 10, the UV emitting lights 101 are predominately arranged to emit light towards a surface of the engagement mechanism 17. As described above, the engagement mechanism 17 may be considered to form part of the aerosol provision device interface of the refill/recharge pack 10 and therefore the UV emitting lights 101 are controlled to emit UV light towards the aerosol provision device interface of the refill/recharge pack 10. This is schematically shown by the arrows in Figure 5c. Therefore, the interface and/or any aerosol-generating material disposed thereon, is exposed to UV light from the UV emitting lights 101, thereby inhibiting or preventing microbial growth on these surfaces.

Although Figure 5c shows the UV emitting lights 101 as being orientated to direct UV light along a direction that is 90° to the longitudinal axis of the receptacle 11 / 21, it should be appreciated that the UV emitting lights 101 may be orientated to emit the UV light along a direction inclined with respect to the longitudinal axis (as described above in Figure 5a). In some implementations, it should also be appreciated that for two or more UV emitting lights 101, the direction along which UV light emitted from the UV emitting lights 101 follows may be different from one another. This may help to, in effect, increase the area of irradiation of UV light on the aerosol provision device interface. It should also be appreciated that the UV emitting lights 101 may be alternatively or additionally arranged to direct UV light along the longitudinal axis of the receptacle 11 / 21. For example, the UV emitting lights 101 may be arranged at the base of the receptacle 21 and arranged to direct UV light towards the protrusion 17a of the engagement mechanism 17 when the aerosol provision device 20 is engaged with the refill/recharge pack 10.

In the context of Figure 5c, the UV emitting lights 101 are controlled to activate (i.e., emit UV light) when the aerosol provision device 20 is located in the receptacle 11. The UV emitting lights 101 may be controlled to activate when it is detected that the aerosol provision device 20 is located in the receptacle 11. For example, a sensor (not shown) in the receptacle 21 may be arranged to sense when the engagement mechanism 17 of the refill/recharge pack 10 is engaged with the aerosol provision device 20. In this case, the UV emitting lights 101 may be controlled by control circuitry 23 of the aerosol provision device 20 and powered by battery 22 of the aerosol provision device 20. Accordingly, when the aerosol provision device 20 determines that it is engaged with the refill/recharge pack 10, the control circuitry 23 is configured to cause the UV emitting lights 101 to be supplied with power from the battery 22 of the aerosol provision device 20 and subsequently generate UV light that is directed towards the engagement mechanism 17.

Hence, more generally, it can be seen that an active sterilisation component, such as one or more UV emitting lights 101, can be implemented in either the refill/recharge pack or the aerosol provision device 20 to provide UV light to one or more of a refill/recharge pack interface of the aerosol provision device 20 and an aerosol provision device interface of the refill/recharge pack 10. In this way, by providing the UV light to one or more of these interfaces, microbial growth can be reduced or prevented. It should also be appreciated that while Figures 5a, 5b and 5c are described as separate implementations, any combination of the arrangements of UV emitting lights 101 as described may be provided in other implementations. For example, in some implementations, the refill/recharge pack 10 may comprise the UV emitting lights 101 of Figure 5a (to irradiate the aerosol provision device interface) and of Figure 5b (to irradiate the refill/recharge pack interface). More generally, any arrangement of UV emitting lights 101 may be provided and the specific arrangement may depend on the degree (or extent) of sterilisation desired for a given implementations.

In both of the implementations described by Figures 5a and 5b, the UV emitting lights 101 are controlled automatically by the control circuitry 13 of the refill/recharge pack 10. For example, the UV emitting lights 101 may be controlled to activate periodically, and in some implementations, the UV emitting lights 101 may alternatively or additionally be controlled to emit UV light when the aerosol provision device 20 is determined as being present or absent in the receptacle 11. In the implementation of Figure 5c, the UV emitting lights 101 are controlled instead by the control circuitry 23 of the aerosol provision device 20. However, in other implementations, the refill/recharge pack 10 and/or the aerosol provision device 20 may be provided with a button or similar user input mechanism, for example on a surface of the housing 10a or 20a, that the user is capable of actuating when the UV emitting lights 101 are activated.

In addition, while the UV emitting lights 101 of Figure 5b have generally been described in the context of emitting UV light towards the refill/recharge pack interface of the aerosol provision device 20, it should be appreciated that in some implementations, the UV emitting lights 101 may additionally be activated when the aerosol provision device 20 is not located in the receptacle 11. In such implementations, the UV emitting light 101 is configured to illuminate the side walls of the receptacle 11. While these side walls may not necessarily receive or be exposed to aerosol-generating material as part of the refilling operation, there may nonetheless be some occurrence of microbial growth on the surfaces of these walls of the receptacle 11. Therefore, illuminating the walls of the receptacle 11 with the UV emitting lights 101 may help to reduce or prevent microbial growth in the receptacle 11. Hence, in some aspects of the present disclosure, there is provided a refilling device (such as refill/recharge pack 10) for refilling an aerosol-generating material storage portion (e.g., reservoir 24) of an aerosol provision device 20 when the aerosol provision device 20 is coupled to the refilling device via a receptacle 11, and a sterilisation component, such as the UV emitting lights 101, configured to prevent or reduce microbial growth at at least one surface of the receptacle 11.

The implementations of Figures 5a to 5c show different implementations that utilise an active sterilisation component. However, implementations in which a passive sterilisation component are used are also contemplated.

Figure 6 schematically represent an implementation of a passive sterilisation component. Figure 6 is based on Figure 4 and similarly shows the lower half of the receptacle 11 including the engagement mechanism 17 and the second and third conduits 16b and 16c (e.g., similarly to that shown in Figures 5a to 5c). It will be appreciated that certain features of Figure 4 have been omitted from Figure 6 for clarity (such as the control circuitry 13, reservoir 14, etc.).

In accordance with the implementation of Figure 6, the passive sterilisation component includes a surface coating 102 (or other surface treatment) of surfaces forming the refill/recharge pack interface of the aerosol provision device 20 and a surface coating 103 of surfaces forming the aerosol provision device interface of the refill/recharge pack 10.

The surface coatings 102, 103 may be any suitable surface coating that is capable of reducing or preventing microbial growth. In some implementations, the surface coating 102, 103 is or comprises silver. However, any suitable surface coating may be used. The surface coating 102, 103 may be applied to an underlying substrate material (for example a plastic or other metal) forming the refill/recharge pack interface of the aerosol provision device 20 and the aerosol provision device interface of the refill/recharge pack 10. The surface coating 102, 103 may be applied in any suitable manner, e.g., chemical or vapour deposition. Alternatively, in some implementations, the substrate forming the refill/recharge pack interface of the aerosol provision device 20 and the aerosol provision device interface of the refill/recharge pack 10 may inherently comprise a surface of an antimicrobial material by virtue of being formed by that material (for example, the engagement mechanism 17 may be formed from silver, and thus comprise a silver surface).

With reference specifically to Figure 6, the surface coatings 102 are applied to surfaces of the aerosol provision device 20 forming the refill/recharge pack interface. For example, as seen in Figure 6, the surface coatings 102 are provided on the base of the receptacle 21 and extend part-way up the vertical walls of the receptacle 21 from the base of the receptacle 21. It should be appreciated that these surfaces of the receptacle 21 may be considered to form a part of the refill/recharge pack interface of the aerosol provision device 20. However, it should also be understood that the surface coatings 102 may be arranged differently from that shown. For example, the surface coatings 102 on the vertical walls of the receptacle 21 may be omitted. Additionally or alternatively, a surface coating 102 may be applied to the reservoir refill mechanism 28 (for example, a passage passing therethrough along which aerosol generating material is capable of flowing). Put more generally, the surface coatings 102 may be arranged on any suitable surface of the aerosol provision device 20 that forms the refill/recharge pack interface.

Similarly, the surface coatings 103 are applied to surfaces of the refill/recharge pack 10 forming the aerosol provision device interface. For example, as seen in Figure 6, the surface coatings 103 are provided on the top surface of the protrusion 17a around the opening to conduit 16c and part-way along the vertical walls of the engagement mechanism 17. It should be appreciated that these surfaces of the engagement mechanism 17 / protrusion 17a may be considered to form a part of the aerosol provision device interface of the refill/recharge pack 10. However, it should also be understood that the surface coatings 103 may be arranged differently from that shown. For example, the surface coatings 103 on the vertical walls of the engagement mechanism 17 may be omitted. Additionally or alternatively, a surface coating 103 may be applied to the inner walls of the conduit 16c. Put more generally, the surface coatings 103 may be arranged on any suitable surface of the refill/recharge pack 10 that forms the aerosol provision device interface.

Accordingly, the surface coatings 102, 103 as shown in Figure 6 act to prevent or reduce microbial growth on the respective surfaces to which they are applied. In this way, similarly to the implementations that use the active sterilisation component, the refill/recharge pack 10 and/or aerosol provision device 20 may be considered relatively more hygienic to users despite the presence of the refill/recharge pack interface of the aerosol provision device 20 and/or the aerosol provision device interface of the refill/recharge pack 10 used for refilling the reservoir 24 of the aerosol provision device 20 which are, at least in some instances, exposed to the environment. By virtue of the surface coatings 102, 103 which, in contrast to the active sterilisation components described above do not require any power or active control to perform the antimicrobial function, a more hygienic interfaces (refill/recharge pack interface of the aerosol provision device 20 and/or the aerosol provision device interface of the refill/recharge pack 10) can be provided.

It should be appreciated that while Figure 6 shows the surface coatings 102 of the aerosol provision device 20 and the surface coatings 103 of the refill/recharge pack 10, in other implementations, only the surface coatings 102 of the aerosol provision device 20 may be implemented, or only the surface coatings 103 of the refill/recharge pack 10 may be implemented.

Hence, more generally, it can be seen that a passive sterilisation component, such as surface coatings 102, 103, can be implemented in either the refill/recharge pack 10 or the aerosol provision device 20 to provide antimicrobial properties to at least one of the refill/recharge pack interface of the aerosol provision device 20 and the aerosol provision device interface of the refill/recharge pack 10. In this way, microbial growth can be reduced or prevented.

It should be appreciated that although the passive sterilisation component (of Figure 6) is described separately to the active sterilisation component (of Figures 5a to 5c), in some implementations, a combination of passive and active sterilisation components may be utilised to provide an overall reduction in microbial growth.

Although Figures 5a to 6 have described sterilisation components that are configured to reduce or prevent microbial growth at one or both of the respective interfaces (i.e., the refill/recharge pack interface of the aerosol provision device 20 and the aerosol provision device interface of the refill/recharge pack 10), it should be appreciated that the sterilisation components may additionally be provided so as to reduce or prevent microbial growth in the respective reservoirs 14, 24 of the aerosol provision device 20 and refill/recharge pack 10.

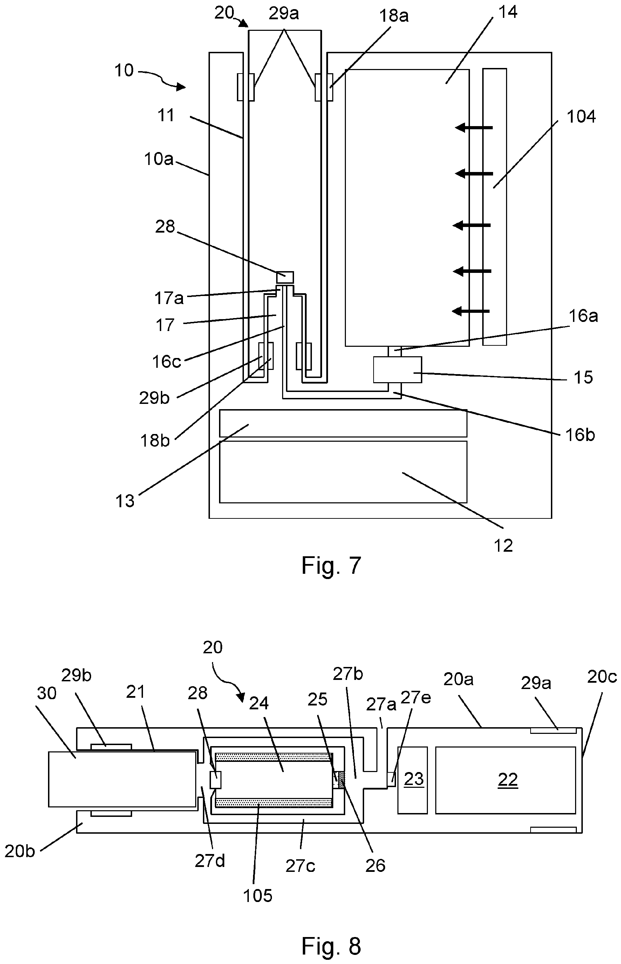

Figure 7 schematically shows an implementation of the refill / recharge pack 10. The refill / recharge pack 10 of Figure 7 is identical to the refill / recharge pack 10 of Figure 4 except a UV emitting light 104 (which may be a single UV emitting light or a plurality of UV emitting lights) is arranged adjacent to the reservoir 14 of the refill / recharge pack 10. In use, the UV emitting light 104 is configured to be activated and emit UV light into the reservoir 14 of the refill/recharge pack 10. In this regard, the reservoir 14 is formed from a material that is transparent to the UV light emitted by the UV emitting light 104. In a similar manner to as described above, the UV light emitted into the reservoir 14 is capable of preventing or reducing microbial growth in the reservoir 14. This may particularly be advantageous when the aerosol-generating material does not comprise nicotine or a similar component with microbial growth inhibiting properties. The UV emitting light 104 may be controlled to activate periodically or in response to a certain condition (e.g., refilling of the aerosol provision device starting/stopping).

Figure 8 schematically shows an implementation of the aerosol provision device 20. The aerosol provision device 20 of Figure 8 is identical to the aerosol provision device 20 of Figure 2 except a surface coating 105 is provided on one or more inner walls of the reservoir 24 of the aerosol provision device 20. In this regard, the surface coating 105 may be formed as any of the surfaces coatings 102 and 103 described with respect to Figure 6. Accordingly, the surface coating 105 in the reservoir 24 is capable of, passively, reducing or preventing microbial growth in the reservoir 24. As above, this may particularly be advantageous when the aerosol-generating material does not comprise nicotine or a similar component with microbial growth inhibiting properties.

It should be appreciated that although Figure 7 shows the UV emitting light 104 for irradiating the reservoir 14 of the refill/recharge pack 10, a similar UV emitting light may be implemented in respect of the reservoir 24 of the aerosol provision device 20. Equally, although Figure 8 shows the surface coating 105 being applied to the inner surface of the reservoir 24, a similar surface coating may be applied to the inner surface of the reservoir 14 of the refill/recharge pack 10. In the described implementation, the refill/recharge pack 10 is configured to both refill the reservoir 24 of the aerosol provision device 20 and to recharge the battery 22 of the aerosol provision device 20. However, in some implementations, the refill/recharge pack 10 may be configured to refill the reservoir 24 of the aerosol provision device 20 only. That is, the recharging circuitry and electrical contacts 29a, 29b may be omitted. Additionally, depending on how the transfer mechanism 15 is operated, and in particular whether the transfer mechanism 15 requires electrical power to operate, the battery 12 of the refill/recharge pack 10 may be omitted. In implementations where the refill/recharge pack 10 is only configured to refill the reservoir 24 of the aerosol provision device 20, the refill/recharge pack 10 may be referred to as a refill pack 10 or refilling device 10.

The configuration of the refill/recharge pack 10 as shown in Figure 4 is to be understood as an example of the refill/recharge pack 10; however, in other implementations, the refill/recharge pack 10 may be configured differently. For example, the position of the electrical contacts 18a, 18b may be different from what is shown in Figure 4. Additionally, the conduit 16, transfer mechanism 15, and engagement mechanism 17 may be different from what is shown. Various aspects of the refill/recharge pack 10 may also depend on the configuration of the aerosol provision device 20 (or vice versa).

In the example shown in Figure 4, the reservoir 14 is located next to the receptacle 11 of the refill/recharge pack 10. This configuration may help achieve overall dimensions of the refill/recharge pack 10 consistent with cigarette packs, as described above. However, it should be appreciated that in other implementations, the position of the reservoir 14 relative to the receptacle 11 may be different from that shown.

In the example shown in Figure 4, the conduit 16 extends from the base of the reservoir 14 and is fed, in effect, in a direction towards the opening of the receptacle 11 when passing along the engagement mechanism 17. However, in other implementations, the conduit 16 and engagement mechanism 17 may be differently configured. For example, if the reservoir refill mechanism 28 is provided on a side of the aerosol provision device 20 / reservoir 24, the engagement mechanism 17 may similarly be provided extending from a side of the receptacle 11. In such implementations, the engagement mechanism 17 may be configured to move between a retracted position (in which the engagement mechanism 17 is moved out of the receptacle 11 therefore not impacting the ability to position the aerosol provision device 20 in the receptacle 11) to an extended position (in which the engagement mechanism 17 is moved into the receptacle 11 to engage with the reservoir refill mechanism 28 of the aerosol provision device 20). The conduit 16 may be arranged accordingly, for example, extending from a side of the reservoir 14. In addition, a moveable engagement mechanism 17 is not limited to the side of the receptacle 11. For example, the engagement mechanism 17 as shown in Figure 4 may alternatively be configured to extend / retract.

In the example shown in Figure 4, the aerosol provision device 20 is inserted with the distal end 20c protruding from the receptacle 11 (or otherwise arranged near the opening of the receptacle 11). In this orientation, the inlet to the reservoir 24 of the aerosol provision device 20 (comprising the reservoir refill mechanism 28) is closer to the engagement mechanism 17 than the aerosol generator 26. In other words, if one assumes that the surface of the refill/recharge pack 10 comprising the opening to the receptacle 11 is a top surface, the aerosol generator 26 is closer to the top surface than the reservoir refill mechanism 28. During refilling, when aerosol-generating material is passed into the reservoir 24 via the reservoir refill mechanism 28, any air that is located in the reservoir 24 is displaced by the aerosol-generating material from the refill/recharge pack 10 via the aerosol generator 26 / aerosol-generating material transport element 25. That is to say, the displaced air may exit the reservoir 24 by following a similar pathway that the aerosol-generating material would otherwise follow in normal operation from the reservoir 24 to the aerosol generator 26. This configuration means that the pressure within the reservoir 24 can be equalised and refilling can be performed uninhibited without provision of a separate air release valve or the like provided in the reservoir 24. In addition, it should be noted that during use of the aerosol provision system, the consumable 30 when installed in the receptacle 21 acts to block or obscure from sight the reservoir refill mechanism 28, thereby providing a sleeker visual appearance to the user.

However, it should be appreciated that in other implementations, the refill/recharge pack 10 and/or aerosol provision device 20 may be configured differently. In some implementations, the reservoir 24 may be provided with an air release valve.

In the described implementation, the engagement mechanism 17 acts dually to function as a mechanism for refilling the reservoir 24 of the aerosol provision device 20 and as a mechanism for recharging the battery 22 of the aerosol provision device 20. However, it should be appreciated that in other implementations, the engagement mechanism 17 may be configured to perform only one of these functions, with the other function being implemented using different components and/or a second engagement mechanism.

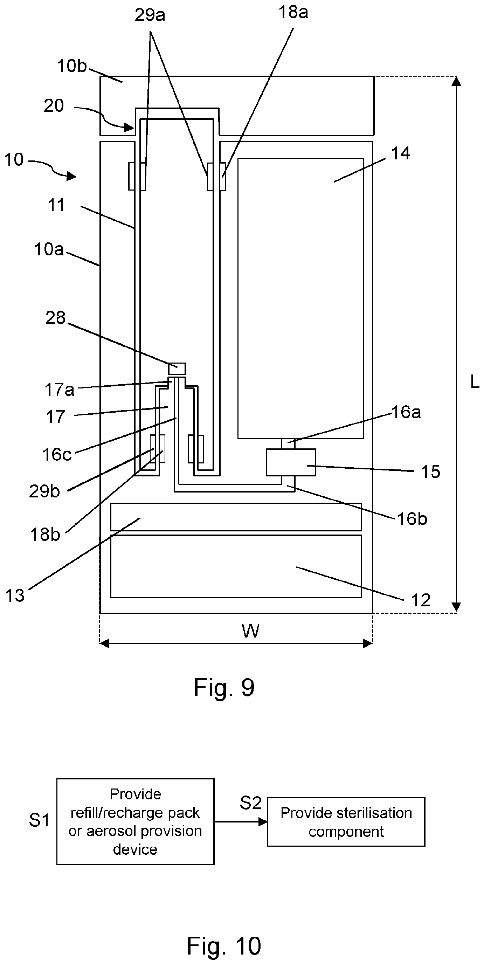

Figure 9 schematically shows a modification of the refill/recharge pack 10 of Figure 4. Figure 9 will be understood from Figure 4, and like components are provided with like reference signs. A description thereof is omitted for conscience.

In Figure 9, the refill/recharge pack 10 comprises a lid 10b which selectively allows access to receptacle 11 when the lid 10b is opened or removed. In some implementations, the lid 10b is a separately component to the housing 10a, that may be removed and coupled to the housing 10a. In other implementations, the lid 10b is movably mounted to the housing 10a of the refill/recharge pack 10. In particular implementations, the lid 10b is arranged to rotate about an axis parallel to the width direction in a hinge-like manner. The lid 10b is capable of moving between a closed position in which the opening of the receptacle 11 is obscured by the lid 10b, and an open position in which the opening of the receptacle 11 is exposed and an aerosol provision device 20 is able to be inserted therein. Cigarette packs often comprise a lid, and thus providing lid 10b on the refill/recharge pack 10 provides increased familiarity to users transitioning to non-combustible aerosol provision systems. As seen in Figure 6, when lid 10b is present, the overall length dimension of the refill/recharge pack 10 includes the extent of the lid 10b in the longitudinal direction.

Figure 10 is a flow diagram representing an example method of reducing or preventing microbial growth at at least one of the refill/recharge interface of an aerosol provision device 20 and the aerosol provision device interface of refill/recharge pack 10.

The method starts at step S1, where either (or both) of an aerosol provision device 20 and a refill/recharge pack 10 are provided.

At step S2, the method provides a sterilisation component at at least one of the refill/recharge pack interface and the aerosol provision device interface. As described above, the sterilisation component may be an active sterilisation component and/or a passive sterilisation component. Suitable techniques for providing the sterilisation component at step S2 may be implemented according to the type of sterilisation component. That is, for a surface coating, suitable techniques for applying the surface coating to a particular surface of the aerosol provision device 20 and/or the refill/recharge pack 10 may be implemented accordingly.

Although not shown in Figure 10, for an active sterilisation component, the method may further include implementing a control step to actively control the sterilisation component to perform a sterilisation effect (such as generating UV light). As described above, such a control step may be implemented in a number of ways depending on the application at hand.

In accordance with the principles of the present disclosure, there is also provided a system including: aerosol provision means (including aerosol provision device 20) comprising an aerosol-generating material storage means (including reservoir 24) for storing an aerosol-generating material, the aerosol provision means arranged to aerosolise aerosol-generating material stored in the aerosol-generating material storage means, and refill interface means (including reservoir refill mechanism 28 and receptacle 21) fluidly coupled to the aerosol-generating material storage means; refilling means (including refill/recharge pack 10) comprising transfer means (including transfer mechanism 15) for transferring aerosol-generating material from the refilling means to the aerosol-generating material storage means when the aerosol provision means is coupled to the refilling means, and an aerosol provision device interface means (including conduit 16c and protrusion 17a) fluidly coupled to a source of aerosol-generating material of the refilling means; and sterilisation means (including UV lights 101, 104, coatings 102, 103, 105). The aerosol provision means is configured to couple to the refilling means via engagement of the refill device interface means with the aerosol provision device interface means such that aerosol-generating material is capable of being transferred to the aerosol-generating material storage means from the refilling means via the coupled interface means. The sterilisation means is configured to prevent or reduce microbial growth at at least one of the refill device interface means and the aerosol provision device interface means.

Thus, there has been described a system including: an aerosol provision device comprising an aerosol-generating material storage portion for storing an aerosol-generating material, the aerosol provision device arranged to aerosolise aerosol-generating material stored in the aerosol-generating material storage portion, and a refill device interface fluidly coupled to the aerosol-generating material storage portion; a refilling device comprising a transfer mechanism for transferring aerosol-generating material from the refilling device to the aerosol-generating material storage portion when the aerosol provision device is coupled to the refilling device, and an aerosol provision device interface fluidly coupled to a source of aerosol-generating material of the refilling device; and a sterilisation component. The aerosol provision device is configured to couple to the refilling device via engagement of the refill device interface with the aerosol provision device interface such that aerosol-generating material is capable of being transferred to the aerosol-generating material storage portion from the refilling device via the coupled interfaces. The sterilisation component is configured to prevent or reduce microbial growth at at least one of the refill device interface and the aerosol provision device interface. Also described is an aerosol provision device, a refilling device and a method for reducing or preventing microbial growth.

While the above described embodiments have in some respects focussed on some specific example aerosol provision systems, it will be appreciated the same principles can be applied for aerosol provision systems using other technologies. That is to say, the specific manner in which various aspects of the aerosol provision system function are not directly relevant to the principles underlying the examples described herein.

The various embodiments described herein are presented only to assist in understanding and teaching the claimed features. These embodiments are provided as a representative sample of embodiments only, and are not exhaustive and/or exclusive. It is to be understood that advantages, embodiments, examples, functions, features, structures, and/or other aspects described herein are not to be considered limitations on the scope of the invention as defined by the claims or limitations on equivalents to the claims, and that other embodiments may be utilised and modifications may be made without departing from the scope of the claimed invention. Various embodiments of the invention may suitably comprise, consist of, or consist essentially of, appropriate combinations of the disclosed elements, components, features, parts, steps, means, etc, other than those specifically described herein. In addition, this disclosure may include other inventions not presently claimed, but which may be claimed in future.

## Claims

1. A system comprising:
an aerosol provision device comprising an aerosol-generating material storage portion for storing an aerosol-generating material, the aerosol provision device arranged to aerosolise aerosol-generating material stored in the aerosol-generating material storage portion, and a refill device interface fluidly coupled to the aerosol-generating material storage portion;
a refilling device comprising a transfer mechanism for transferring aerosol-generating material from the refilling device to the aerosol-generating material storage portion when the aerosol provision device is coupled to the refilling device, and an aerosol provision device interface fluidly coupled to a source of aerosol-generating material of the refilling device; and
a sterilisation component,
wherein the aerosol provision device is configured to couple to the refilling device via engagement of the refill device interface with the aerosol provision device interface such that aerosol-generating material is capable of being transferred to the aerosol-generating material storage portion from the refilling device via the coupled interfaces, and
wherein the sterilisation component is configured to prevent or reduce microbial growth at at least one of the refill device interface and the aerosol provision device interface.

2. The system of claim 1, wherein the sterilisation component comprises a UV light emitter arranged to direct UV light towards the at least one of the refill device interface and the aerosol provision device interface.

3. The system of claim 2, wherein the UV light emitter is provided as part of the refilling device.

4. The system of claim 2 or 3, wherein the refilling device comprises an aerosol provision device receiving section configured to receive at least a part of the aerosol provision device therein, and wherein the UV light emitter is arranged to direct light into the aerosol provision device receiving section.

5. The system of claim 4, wherein the UV light emitter is configured to be operated in at least one of:
when the aerosol provision device is provided in the aerosol provision device receiving section;
when the refilling device is controlled to perform a refilling operation; and
when the aerosol provision device is removed from the aerosol provision device receiving section.

6. The system of any of claims 2 to 5, wherein the UV light emitter is configured to be operated when a user engages with a UV light emitter activation button provided on at least one of the refilling device and the aerosol provision device.

7. The system of claim 1, wherein the sterilisation component comprises a coating, the coating provided on at least one of the refill device interface and the aerosol provision device interface.

8. The system of claim 7, wherein the coating is an anti-microbial growth coating, and wherein optionally the coating is, or comprises, silver.

9. The system of any of the preceding claims, wherein the sterilisation component is further configured to prevent or reduce microbial growth at or in the aerosol-generating material storage portion of the aerosol provision device.

10. The system of any of the preceding claims, wherein the sterilisation component is further configured to prevent or reduce microbial growth at or in an aerosol-generating material storage portion of the refilling device.

11. The system of any of the preceding claims, wherein the system further comprises aerosol-generating material that is free from active ingredients and / or flavourants.

12. An aerosol provision device for aerosolising aerosol-generating material, the aerosol provision device comprising:
an aerosol-generating material storage portion for storing an aerosol-generating material,
a refill device interface fluidly coupled to the aerosol-generating material storage portion and arranged to engage with an aerosol provision device interface of a refilling device, the refilling device comprising a transfer mechanism for transferring aerosol-generating material from the refilling device to the aerosol-generating material storage portion when the aerosol provision device is coupled to the refilling device; and
a sterilisation component,
wherein the aerosol provision device is configured to couple to the refilling device via engagement of the refill device interface with the aerosol provision device interface such that aerosol-generating material is capable of being transferred to the aerosol-generating material storage portion from the refilling device via the coupled interfaces, and
wherein the sterilisation component is configured to prevent or reduce microbial growth at least the refill device interface.

13. A refilling device for refilling an aerosol-generating material storage portion of an aerosol provision device arranged to aerosolise aerosol-generating material stored in the aerosol-generating material storage portion, the refilling device comprising:
a transfer mechanism for transferring aerosol-generating material from the refilling device to the aerosol-generating material storage portion when the aerosol provision device is coupled to the refilling device,
an aerosol provision device interface fluidly coupled to a source of aerosol-generating material of the refilling device and configured to engage with a refill device interface of the aerosol provision device; and
a sterilisation component,
wherein the refilling device is configured to couple to the aerosol provision device via engagement of the aerosol provision device interface with the refill device interface such that aerosol-generating material is capable of being transferred to the aerosol-generating material storage portion from the refilling device via the coupled interfaces, and
wherein the sterilisation component is configured to prevent or reduce microbial growth at at least the aerosol provision device interface.

14. A method for reducing or preventing microbial growth at at least one of a refill device interface of an aerosol provision device and the aerosol provision device interface of a refilling device, wherein the refilling device is configured to refill an aerosol-generating material storage portion of the aerosol provision device with aerosol generating material via engagement of the refill device interface with the aerosol provision device interface, the method comprising:
providing a sterilisation component at at least one of the refill device interface and the aerosol provision device interface.

15. A system comprising:
aerosol provision means comprising an aerosol-generating material storage means for storing an aerosol-generating material, the aerosol provision means arranged to aerosolise aerosol-generating material stored in the aerosol-generating material storage means, and refill interface means fluidly coupled to the aerosol-generating material storage means;
refilling means comprising transfer means for transferring aerosol-generating material from the refilling means to the aerosol-generating material storage means when the aerosol provision means is coupled to the refilling means, and an aerosol provision device interface means fluidly coupled to a source of aerosol-generating material of the refilling means; and
sterilisation means,
wherein the aerosol provision means is configured to couple to the refilling means via engagement of the refill device interface means with the aerosol provision device interface means such that aerosol-generating material is capable of being transferred to the aerosol-generating material storage means from the refilling means via the coupled interface means, and
wherein the sterilisation means is configured to prevent or reduce microbial growth at at least one of the refill device interface means and the aerosol provision device interface means.
